# EUROPÄISCHE PATENTSCHRIFT

(11) **EP 0 664 919 B1**
(45) Veröffentlichungstag und Bekanntmachung des Hinweises auf die Patenterteilung: **14.05.1997**
(21) Anmeldenummer: 93923479.5
(22) Anmeldetag: 16.10.1993
(51) Int. Cl.: G11B 20/00, G11B 23/36, H04N 5/44, H04B 1/06, A63J 17/00, A61M 21/00, A61L 9/12

(54) **AUFZEICHNUNGSTRÄGER UND GERÄT ZUM ERZEUGEN VON TÖNEN UND/ODER BILDERN**
RECORDING MEDIUM AND APPLIANCE FOR GENERATING SOUNDS AND/OR IMAGES
SUPPORT D'ENREGISTREMENT ET APPAREIL POUR PRODUIRE DES SONS ET/OU DES IMAGES

(30) Priorität: 16.10.1992 DE 4234926; 19.02.1993 DE 4305141; 22.04.1993 DE 4313119
(43) Veröffentlichungstag der Anmeldung: 02.08.1995
(73) Patentinhaber: TEBBE, Gerold, MC-9800 Monaco (MC)
(72) Erfinder: TEBBE, Gerold, MC-9800 Monaco (MC)
(74) Vertreter: Ostertag, Reinhard
(86) Internationale Anmeldenummer: EP9302858
(87) Internationale Veröffentlichungsnummer: WO9409493

(56) Entgegenhaltungen:
- EP-A- 0 359 325
- EP-A- 0 508 939
- DE-A- 4 003 476
- DE-A- 4 003 477
- DE-C- 4 033 076
- FR-A- 2 670 568
- US-A- 4 629 604
- US-A- 4 753 148
- US-A- 4 952 024
- US-A- 5 062 097
- US-A- 5 113 738
- PATENT ABSTRACTS OF JAPAN vol. 16, no. 257 (C-0949)11. Juni 1992 & JP,A,04 058 956 (MATSUSHITA ELECTRIC IND. CO., LTD.) 25. Februar 1992
- PATENT ABSTRACTS OF JAPAN vol. 15, no. 317 (C-0858)13. August 1991 & JP,A,03 121 074 (TECHNO RYOWA : K.K.) 23. Mai 1991
- PATENT ABSTRACTS OF JAPAN vol. 14, no. 134 (C-0701)14. März 1990 & JP,A,02 007 969 (TOSHIBA CORP.) 11. Januar 1990
- PATENT ABSTRACTS OF JAPAN vol. 10, no. 197 (E-418)10. Juli 1986 & JP,A,61 041 229 (TAMAPATSUKU K.K.) 27. Februar 1986
- PATENT ABSTRACTS OF JAPAN vol. 16, no. 180 (C-0935)30. April 1992 & JP,A,04 022 369 (MATSUSHITA ELECTRIC IND. CO., LTD.) 27. Januar 1992
- PATENT ABSTRACTS OF JAPAN vol. 4, no. 56 (E-008)25. April 1980 & JP,A,55 025 276 (UCHIUMI MINORU) 22. Februar 1980
- DATABASE WPI Section EI, Week 8429, Derwent Publications Ltd., London, GB; Class S06, AN 84-177169
- PATENT ABSTRACTS OF JAPAN vol. 15, no. 401 (E-1121)11. Oktober 1991 & JP,A,03 163 796 (BROTHER IND., LTD.) 15. Juli 1991
- PATENT ABSTRACTS OF JAPAN vol. 15, no. 401 (E-1121)11. Oktober 1991 & JP,A,03 163 797 (BROTHER IND., LTD.) 15. Juli 1991
- PATENT ABSTRACTS OF JAPAN vol. 17, no. 471 (P-1601)26. August 1993 & JP,A,05 109 253 (TOSHIBA CORP.) 30. April 1993

## Beschreibung

Die Erfindung betrifft ein Gerät zum Erzeugen von Tönen und/oder Bildern gemäß dem Oberbegriff des Anspruches 1.

Ein derartiges Gerät ist in der US-A-4 629 604 offenbart. Bei ihm umfaßt der Steuersignalgenerator eine Videokassette, auf welcher neben der Ton/Bild-Spur zusätzlich eine Steuerspur vorgesehen ist, durch welche unterschiedliche Duftgeneratoren angesteuert werden. Um Vermischungen der von den verschiedenen Duftgeneratoren abgegebenen Düfte kleinzuhalten, wie sie beim sequentiellen Aktivieren unterschiedlicher Duftquellen entstehen können, wird vorgeschlagen, die Duftstoffe in ähnlich wie ein Docht arbeitenden Duftpolstern zu speichern und aus diesen selektiv durch zugeordnete steuerbare elektrische Heizwiderstände auszutreiben.

In der US-A-5 062 097 ist ein Gerät zum Erzeugen von Tönen und/oder Bildern offenbart, welches mit einer Schnittstelle zu einem externen elektrischen oder elektronischen Musikinstrument versehen ist, über welche dieses Musikinstrument automatisch synchron zur Wiedergabe des Hauptgerätes gespielt wird. Hiezu sind in die Ton/Bilddaten des Aufzeichnungsträgers des Hauptgerätes weitere Steuerdaten eingestreut, die für das Ansteuern des externen Musikinstrumentes verwendet werden.

In der DE-C-4 003 076 ist ein Rundfunk- oder Fernsehgerät offenbart, das unter Benutzung von zwischen die Radio/Bild-Signale eingeschobenen Steuerdaten Duftgeneratoren ansteuert.

In der EP-A-0 508 939 ist ein ähnliches Gerät beschrieben, welches zusätzlich auch noch einen Windgenerator, Kältegenerator und Hitzegenerator ansteuern kann, um Wetterbedingungen nachzubilden.

In der JP-A-4 022 369 (und Zusammenfassung in PAJ) ist ein computergesteuertes Multimedia-Gerät beschrieben, welches neben einem Lautsprecher und einem Monitor auch einen Duftgenerator und einen Vibrator ansteuert.

Die US-A-4 753 148 offenbart ein Multimedia-Gerät, bei welchem auf einem Massenspeicher (Diskette) eine Vielzahl von Steuerprogrammen für eine Lichtorgel abgelegt sind. Musik oder Musikvideos sind auf einem Audioband bzw. einem Videoband abgelegt, und ein Steuerrechner synchronisiert die Steuerung der Lichtorgel auf die jeweils gespielte Musik.

In der DE-A-40 03 477 ist ein Gerät zur gleichzeitigen Stimulation von Augen und Ohren beschrieben. Auf einem Tonband sind sowohl Ton-Nutzsignale als auch Steuersignale für in eine Brille eingebaute Leuchtdioden aufgezeichnet. Beim Abspielen des Aufzeichnungsträgers wird die entsprechende Musik zusammen mit den Licht-Steuersignalen entsprechenden Lichtimpulsen abgegeben.

In der FR-A-2 670 568 ist ein Gerät zur Luftverbesserung in Räumen beschrieben, bei dem aus einer Mehrzahl von Vorratsbehältern Duftstoffe in die aufzubereitende Luft eingespeist werden können. Die Duftstoffzugabe erfolgt gesteuert durch Magnetventile von einem Bedienfeld her.

In der JP-A-2 007 969 (und Zusammenfassung in PAJ) ist ein Duftgenerator beschrieben, der durch ein fortlaufendes Band gebildet ist, auf welchem sich zwei oder mehr verschiedene mikroverkapselte Aromastoffe befinden. Durch selektives Zerstören der unterschiedliche Aromastoffe enthaltenden Mikrokapseln kann man gesteuert unterschiedliche Düfte erzeugen.

In der JP-A-55025276 (und Zusammenfassung in PAJ) ist ein Stereo-Bildtelefon beschrieben, welches zugleich einen elektronischen Duftstoff-Verteiler enthält.

Herkömmliche Gerät zum Erzeugen von Tönen oder Bildern erzeugen sich verhältnismäßig schnell ändernde akustische und/oder visuelle Reize. Die schnellen Änderungen der Reize entsprechen dem durchschnittlichen, hohen Informations-Aufnahmevermögen des Menschen und sind für klassische Tonwerke, Sprachwerke und Wiedergabe von Theateraufführungen oder Wiedergabe von Filmen durch die Vorgaben der Autoren oder Künstler im wesentlichen festgelegt.

Wo Ton- oder Bildwiedergaben von einem Zuhörer oder Zuschauer für sein persönliches Interesse zur Entspannung zu Hause abgespielt werden, kommt es auf eine absolute Werktreue nicht immer an. Erfindungsgemäß kann man für diesen Einsatz einen zusätzlichen entspannenden Effekt dadurch erzielen, daß man in dem die Töne und/oder Bilder erzeugenden Gerät zusätzlich eine Steuersignalquelle vorsieht, die verglichen mit den akustischen und/oder visuellen Reizen nur langsam veränderliche Steuersignale bereitstellt, durch welche ein zusätzlicher Reizgenerator aktivierbar ist. Dieser zusätzliche Reizgenerator kann visuelle, taktile oder sensorische Reize erzeugen oder auch ein weiterer akustischer Reizgenerator sein. Durch die zusätzlichen langsam veränderlichen Reize erhält man eine entspannende und beruhigende oder auch eine stimulierende Wirkung beim Zuhörer oder Betrachter, je nach Art des verwendeten Reizgenerators.

Ein erfindungsgemäßes Abspielgerät gemäß Anspruch 1 unterscheidet sich von einem herkömmlichen Abspielgerät nur durch den zusätzlich vorgesehenen Steuersignalspeicher, einige zum Auslesen von Daten aus dem Steuersignalspeicher benötigte Schaltkreise sowie ggf. eine etwas andere Programmierung einer zentralen Steuereinheit des Gerätes. In den wesentlichen Teilen bleibt das Gerät unverändert. Ein erfindungsgemäßes Gerät läßt sich somit mit nur geringen Mehrkosten gegenüber einem herkömmlichen Gerät herstellen.

Dabei ist bei dem erfindungsgemäßen Gerät gewährleistet, daß das Ansteuern der verschiedenen Reizgeneratoren unter weitestgehender Entlastung der Steuereinheit erfolgt.

Vorteilhafte Weiterbildungen der Erfindung sind in Unteransprüchen angegeben.

Mit einem Gerät gemäß Anspruch 2 kann man das durch die Wiedergabe der Ton- und/oder Bildsignale und die zusätzlichen Reize erhaltene Gesamterlebnis in weitem Rahmen und sehr abwechselungsreich variieren. So kann man insbesondere sensorische Reize, die sich nur mit großer Zeitkonstante ändern lassen, koppeln mit etwas rascheren, verglichen mit der Ton- und/oder Bildwiedergabe aber immer noch langsamen Reizen, z. B. Änderungen in der Helligkeit des Raumes, Änderungen in der Stellung der Lehne und Sitzfläche eines Sessels usw..

Gemäß Anspruch 3 kann man von den in Rundfunksignalen oder Fernsehsignalen enthaltenen frequenzmäßigen oder zeitlichen Lücken davon Gebrauch machen, Steuersignale zur Ansteuerung von Reizgeneratoren einzustreuen.

Gemäß Anspruch 4 werden Randbereiche oder Zwischenbereiche von bespielten Aufzeichnungsträgern für Ton und/oder Bild für das Anbringen von Reizgenerator-Steuersignalen verwendet.

Gemäß Anspruch 6 kann man die erfindungsgemäßen Vorteile auch in Verbindung mit herkömmlichen bespielten Aufzeichnungsträgern erhalten. Ein Eingriff in das Abspielgerät ist hierbei nicht notwendig.

Mit der Weiterbildung der Erfindung gemäß Anspruch 8 wird erreicht, daß die Kopplung zwischen Lichterzeugung und Klangerzeugung mit sehr einfachen apparativen Mitteln vorgenommen werden kann, auch bei schon vorhandenen Ton-Wiedergabegeräten. Der Filterkreis kann dann entweder auf niedere Frequenzen ausgelegt sein, so daß man dann das Lichtsignal vom Grundtakt des Musikstückes ableitet. Statt dessen kann man auch in das aufgezeichnete Musikstück einen oder mehrere Pilottöne hineinmischen, die dann über den Filterkreis bei der Wiedergabe abgetrennt und zur Steuerung des Reizgenerators verwendet werden.

Gemäß Anspruch 9 erhält man die durch die Kopplung von Wiedergabe und Reizgenerator erhaltenen Vorteile auch bei einem ad hoc auf einem elektronischen Musikgerät gespielten Musikstück.

Die Weiterbildung der Erfindung gemäß Anspruch 10 gestattet es, die Frequenz des Steuersignals unabhängig vom Nutzsignal einzustellen, wobei aber eine Phasenkopplung zwischen beiden Signalen erhalten bleibt.

Bei einem Gerät gemäß Anspruch 11 kann man der durch Ankopplung an das Nutzsignal gegebenen Steuerung des Reizgenerators zusätzlich noch eine sich frei ändernde Modulation überlagern. So kann der Hüllkurvengenerator z. B. die Amplitude des Steuersignales gemäß einer Sinuskurve mit sehr niedriger Frequenz variieren oder gemäß einem z. B. in einem ROM abgelegten digitalen Programm variieren.

Bei den bisher üblichen Geräten zur Erzeugung von Tönen und/oder Bildern waren sensorische Reize ausgeschlossen. Mit einem Gerät gemäß Anspruch 12 eröffnet sich insgesamt eine neue Dimension entspannender Wiedergabe von Ton und/oder Bild, die in Zukunft auch für gezielte Multisimulations-Kompositionen ausgenützt werden können.

Ein Gerät gemäß Anspruch 13 eignet sich zur einfachen und effektiven gesteuerten Freigabe von in flüssiger Form vorliegenden Duftstoffen.

Mit der Weiterbildung der Erfindung gemäß Anspruch 14 wird erreicht, daß die Freisetzung von Duftstoffen mit einer Abgabe von zusätzlichem Sauerstoff einhergeht.

Verwendet man gemäß Anspruch 15 mikroverkapselte Duftstoffe, so ist die Lagerung, der Versand und die Handhabung der Duftstoffe besonders einfach. Man hat auch bei längerem Stillstand des Gerätes nur einen geringen Duftstoffverlust. Die steuerbare Duftquelle kann kompakt bauend, mechanisch einfach und preisgünstig hergestellt werden.

Dies gilt insbesondere für eine Duftstoffquelle, wie sie im Anspruch 16 angegeben ist, da über die Winkelgeschwindigkeit der Quetschwalzen auf sehr einfache Weise die Duftabgaberate einstellbar ist.

Ein Duftgenerator, wie er im Anspruch 17 angegeben ist, zeichnet sich durch besonders niedere Betriebskosten aus. Er hat auch einen mechanisch noch einfachen Aufbau, so daß er sich auch als preisgünstiges Massenprodukt eignet.

Die Weiterbildung der Erfindung gemäß Anspruch 18 stellt sicher, daß durch die Steuersignale genau die gewünschten Duftabgaben erhalten werden, da der Behälterblock als eine Einheit angeschlossen wird, also Fehlverbindungen ausgeschlossen sind.

Die Weiterbildung der Erfindung gemäß Anspruch 19 ist im Hinblick auf ein rasches Überstellen der Duftstoffe vom Duftgenerator zum Benutzer von Vorteil.

Bei einem Duftgenerator gemäß Anspruch 20 ist der Anwender nicht mit Vorbereitungsarbeiten belastet. Die mikroverkapselten Duftstoffe tragenden Substrate liegen als anwendungsfertige Produkte vor.

Die Weiterbildung der Erfindung gemäß Anspruch 21 ist im Hinblick auf ein gezieltes unterschiedliches Freisetzen von Duftstoffen unterschiedlicher Art von Vorteil.

Verwendet man zum Aufbrechen der Mikrokapseln gemäß Anspruch 22 einen Ultraschallgenerator, so erfolgt dieses Aufbrechen sehr wirksam; die Kapseln können also von Hause aus verhältnismäßig stabil sein, was die Lagerung und Handhabung vor der Verwendung vereinfacht.

Auch die Weiterbildung der Erfindung gemäß Anspruch 23 und 24 ist im Hinblick auf ein effektives Aufbrechen der Mikrokapseln von Vorteil.

Bei einem Abspielgerät gemäß Anspruch 25 erfolgt das Aufbrechen der Mikrokapseln in einem bestimmten Bereich des Substrates immer vollständig, und die Steuerung der Duftintensität erfolgt über den Vorschub des die Mikrokapseln tragenden Substrates. Dies ist im Hinblick auf zuverlässiges Freisetzen der Duftstoffe bei gleichzeitiger feiner Dosierbarkeit der Duftintensität von Vorteil.

Bei einem Gerät gemäß Anspruch 26 erhält man ein akustisches Spiegelbild der Lichterzeugung.

Koppelt man die Lichtsteuereinheit über ein akustisches oder optisches (z. B. IR-) Modem an ein Wiedergabegerät an, wie im Anspruch 27 angegeben, so erhält man eine phasenrichtige Erzeugung von zusätzlichen Reizen ohne Eingriffe in das Wiedergabegerät. Man kann dann diese zusätzliche Einheit als in sich autarke Einheit vermarkten, die auch von technisch weniger begabten Verbrauchern problemlos in Betrieb genommen werden kann.

Dies ist bei Zusammenfassen der verschiedenen Teileinheiten in einem gemeinsamen Gehäuse gemäß Anspruch 28 nochmals vereinfacht.

Die Weiterbildung der Erfindung gemäß Anspruch 29 gestattet die Erzeugung von zusätzlichen optischen Reizen, die in weiten Grenzen farbmäßig, mustermäßig und intensitätsmäßig variiert werden können, unter Verwendung des in den meisten Haushalten sowieso vorhandenen Fernsehgerätes.

Nachstehend wird die Erfindung anhand von Ausführungsbeispielen unter Bezugnahme auf die Zeichnung näher erläutert. In dieser zeigen:
- Figur 1:: ein Blockschaltbild eines elektronischen Tasteninstrumentes, welches zusätzlich mit einer Einrichtung von zur Begleitmusik synchronen, jedoch höher frequenten visuellen Reizen versehen ist;
- Figur 2:: ein Blockschaltbild eines CD-Wiedergabegerätes, welches zusätzlich mit einer Einrichtung zur phasen-synchronisierten Erzeugung höherfrequenter visueller Reize versehen ist;
- Figur 3:: ein ähnliches Blockschaltbild wie Figur 2, in welchem jedoch ein abgewandeltes CD-Abspielgerät wiedergegeben ist, bei welchem zugleich eine Einrichtung zur kontrollierten Abgabe von Duftstoffen vorgesehen ist;
- Figur 4:: ein ähnliches Blockschaltbild wie Figur 2, in welchem ein nochmals abgewandeltes Entspannungsgerät wiedergegeben ist;
- Figur 5:: eine schematische Darstellung einer steuerbaren Duftquelle, wie sie bei dem Entspannungsgerät nach Figur 4 verwendet wird, wobei eine Seitenplatte eines Geräterahmens weggebrochen ist;
- Figur 6:: ein Blockschaltbild eines Abspielgerätes für Compact-Discs zusammen mit einer Multistimulations-Compact-Disc;
- Figur 7:: einen Ausschnitt aus einer abgewandelten Multistimulations-Compact-Disc;
- Figur 8:: eine schematische Ansicht eines Ausschnittes aus einem Multistimulations-Magnetband;
- Figur 9:: eine schematische Aufsicht auf ein abgewandeltes Multistimulations-Magnetband;
- Figur 10:: eine schematische Darstellung eines steuerbaren Duftgenerators für drei verschiedene Duftstoffe;
- Figur 11:: einen schematischen vergrößerten Schnitt durch ein Duftstoff-Trägerband;
- Figur 12:: eine schematische Darstellung eines Duftstoffgenerators, der zusammen mit einem Duftstoff-Trägerband gemäß Figur 6 verwendbar ist;
- Figur 13:: eine ähnliche Darstellung wie Figur 6, wobei eine normale Compact-Disc Verwendung findet und eine gesonderte Eingabeeinrichtung für Reizgenerator-Steuersignale vorgesehen ist;
- Figur 14:: ein Blockschaltbild eines Fernsehgerätes, welches mit einer Zusatzeinrichtung zum Erzeugen langsam veränderlicher sensorischer, taktiler und anderer Reize versehen ist;
- Figur 15:: einen schematisch veranschaulichten Ausschnitt aus dem digitalen Datenstrom eines Aufzeichnungsträgers für ein digitalisiertes Tonsignal;
- Figur 16:: einen schematisch veranschaulichten Datenblock des Datenstroms nach Figur 15;
- Figur 17:: ein Blockschaltbild eines Decoders für Datenblöcke gemäß Figur 16; und
- Figur 18:: ein Blockschaltbild eines Ausführungsbeispiels für ein Abspielgerät mit dem Decoder nach Figur 17.

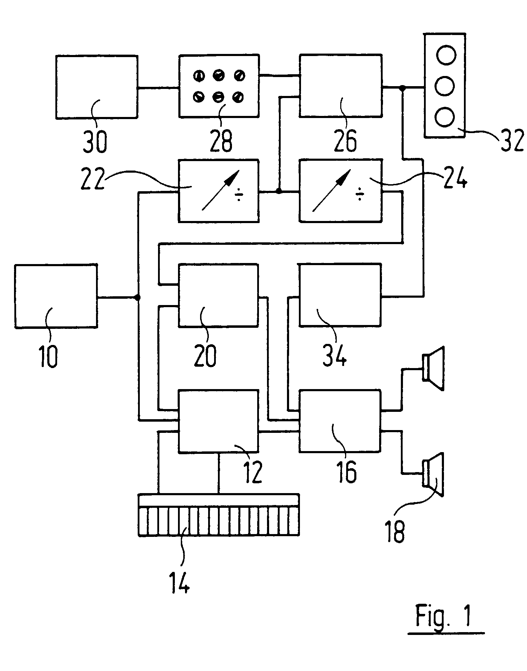

In Figur 1 ist mit 10 ein zentraler Taktgeber für ein Keyboard bezeichnet. Dieser stellt die Ausgangsfrequenz für einen Tongenerator 12 bereit, der seinerseits mit einem Tastenfeld 14 zusammenarbeitet. Dieses ist mit zusätzlichen Tasten oder Knöpfen zum Einstellen von Begleitmusik und/oder Instrumentenart versehen, wie an sich bekannt.

Der Ausgang des Tongenerators 12 ist mit einem der Eingänge eines Stereo-Mischverstärkers 16 verbunden, der Lautsprecher 18 ansteuert.

Ein zweiter Eingang des Mischverstärkers 16 ist mit dem Ausgang eines Rhythmusgenerators 20 verbunden, der zwei Eingänge aufweist. Ein erster Eingang erhält ein die Rhythmusart vorgebendes Steuersignal, das vom Tastenfeld 14 her bereitgestellt wird, sowie ein die Rhythmus-Taktfrequenz vorgebendes zweites Steuersignal, das von zwei hintereinandergeschalteten Frequenzteilern 22, 24 aus dem Ausgangssignal des Taktgebers 10 abgeleitet wird. Die beiden Frequenzteiler 22, 24 haben ein einstellbares Teilverhältnis, wie durch Pfeile angedeutet.

Die Verbindungsleitung zwischen den beiden Frequenzteilern 22, 24 ist mit dem einen Eingang eines Licht-Steuerkreises 26 verbunden. Dieser erhält an seinem zweiten Eingang Steuersignale, welche die Amplitude und Farbe des zu erzeugenden Lichtes vorgeben und von einem Einstellkreis 28 bereitgestellt werden. Dieser erhält seinerseits ein sich nach einem vorgegebenen Muster ändernde Rohsignal, das von einem Signalgenerator 30 bereitgestellt wird. Hierbei kann es sich um einen mit kleiner Frequenz laufenden Sinusgenerator oder um einen digital arbeitenden Spannungsgenerator handeln, der z.B. in einem ROM abgelegt eine sich langsam ändernde Tonsignal-Hüllkurve enthält.

Entsprechend den auf seine Eingänge gegebenen Steuersignalen erzeugt der Licht-Steuerkreis 26 Lichtsignale für einen Ein- oder Mehrfarben-Lichterzeuger 32, wobei deren Frequenz vom Ausgangssignal des Frequenzteilers 22 vorgegeben ist, deren Amplitude durch den Einstellkreis 28 und den Signalgenerator 30 vorgegeben ist.

Man erkennt, daß bei dem oben beschriebenen elektronischen Keyboard synchron zur Begleitmusik, deren Takt am Frequenzteiler 22 und 24 eingestellt wird, eine gegenüber dem Rhythmustakt höherfrequente Erzeugung von Lichtimpulsen erhalten wird, wobei das Verhältnis zwischen der Frequenz der Lichtimpulse und dem Takt der Begleitmusik am Frequenzteiler 24 eingestellt werden kann.

Zusätzlich ist bei dem Keyboard nach Figur 1 vorgesehen, die Lichtsignale zur Ansteuerung eines Klanggenerators 34 zu verwenden, der z.B. im einfachsten Fall drei entsprechend gesteuerte Sinusimpulsfolgen unterschiedlicher Höhe bereitstellt. Der Klanggenerator 34 kann aber auch komplizierteren Aufbau aufweisen, z.B. Klangsignale mit breiterem Spektrum in Abhängigkeit von den Lichtsignalen bereitstellen, die z.B. Triangeln, Holztrommeln oder dergleichen wiedergeben. Das Ausgangssignal des Klanggenerators 34 wird auf einen dritten Eingang des Mischverstärkers 16 gegeben, so daß die vom Lichterzeuger 32 erzeugten Lichtimpulse ein akustisches Spiegelbild haben.

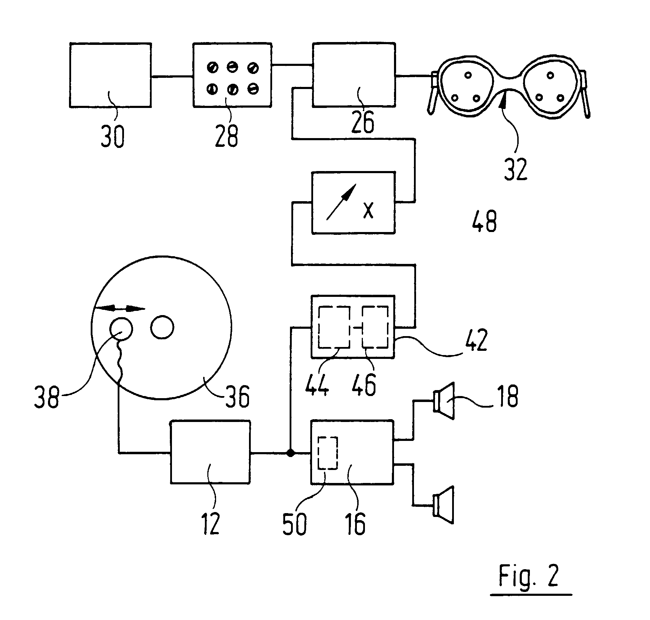

In Figur 2 ist ein CD-Wiedergabegerät schematisch dargestellt, welches ebenfalls mit einer Einrichtung zur phasenrichtigen Erzeugung höherfrequenter Lichtimpulse versehen ist.

Eine Compact Disc 36 wird von einem nicht wiedergegebenen Antrieb gedreht. Ihre Aufzeichnungsspuren werden von einem Abtastkopf 38 ausgelesen, der durch einen ebenfalls nicht wiedergegebenen Antrieb in radialer Richtung verfahrbar ist.

Durch das Ausgangssignal des Abtastkopfes 38 wird nun der Tongenerator 12 angesteuert, dessen Ausgangssignal wieder über den Verstärker 16 auf die Lautsprecher 18 abgegeben wird.

Das Ausgangssignal des Tongenerators 12 wird auf einen speziellen Signalformkreis 42 gegeben, den man z.B. durch Hintereinanderschalten eines Filters 44 und eines durch dessen Ausgangssignal angesteuerten Schmidt-Triggers 46 realisieren kann. Ist das Filter 44 auf niedere Frequenzen eingestellt, erhält man am Ausgang des Schmidt-Triggers 46 Impulse mit der Frequenz der niederfrequenten Begleitmusik des vom Abtastkopf 38 ausgelesenen Musikstückes.

Das Ausgangssignal des Signalformkreises 42 wird über einen einstellbaren Frequenzvervielfacher 48 wieder auf den einen Eingang des Licht-Steuerkreises 26 gegeben. Dessen zweiter Eingang erhält wieder das vom Einstellkreis 28 modifizierte Ausgangssignal des Signalgenerators 30. Der Lichterzeuger 32 ist nun als Brille wiedergegeben, die auf undurchsichtigen Gläsern jeweils drei Leuchtdioden für die drei Grundfarben aufweist. Wahlweise kann man wiederum nur Licht einer einzigen Farbe erzeugen und hierfür nur eine Leuchtdiode oder mehrere bei gleicher Wellenlänge arbeitende Leuchtdioden verwenden.

Damit erhält man ohne nennenswerten Eingriff in das CD-Wiedergabegerät eine Erzeugung von Lichtimpulsen, die phasenrichtig zur Begleitmusik eines Musikstückes erfolgt, jedoch gegenüber dem Takt des Musikstückes einstellbare, in der Regel höhere Frequenz aufweist.

In Abwandlung des oben beschriebenen Ausführungsbeispieles kann man bei der Herstellung der Compact Disc bzw. Schallplatte oder Musikkassette eine Mischstufe verwenden, deren einer Eingang mit dem Musik-Tonsignal beaufschlagt ist, während ihr anderer Eingang reine Sinussignale erhält, welche gemäß den Steuerbefehlen für den Lichterzeuger moduliert sind. Die so mit aufgezeichneten Steuersignale werden dann vom entsprechend abgestimmten Signalformkreis 42 wieder verwendet. Gleichzeitig sind diese Steuersignale in den Lautsprechern 18 hörbar, so daß man wiederum simultane visuelle und akustische Reize erhält. Wird letzteres nicht gewünscht, kann man am Eingang des Verstärkers 16 einen Sperrkreis 50 vorsehen, der die sinusförmigen Licht-Steuersignale abblockt.

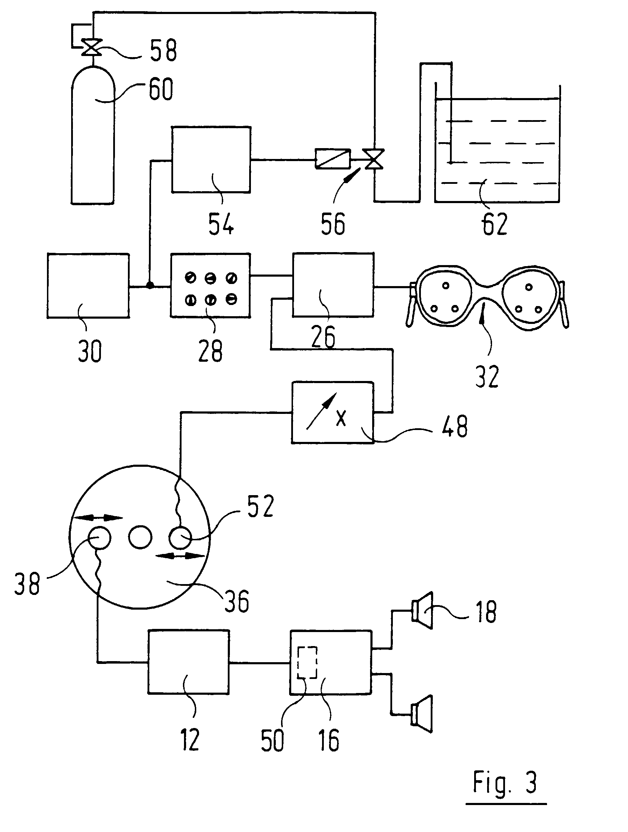

Beim Ausführungsbeispiel nach Figur 3 sind wiederum Geräteteile, die obenstehend schon beschrieben wurden, mit denselben Bezugszeichen versehen und werden nicht nochmals im einzelnen beschrieben.

Auf der Compact Disc 36 sind nun die Steuersignale für den Lichterzeuger 32 auf einer gesonderten Aufzeichnungsspur abgelegt, und diese wird durch einen zweiten Abtastkopf 52 ausgelesen. Der Ausgang des Abtastkopfes 52 ist entweder direkt mit dem Eingang des Frequenz-Vervielfachers 48 verbunden, wie dargestellt, oder bei höherfrequenter Wahl des Steuersignales auch direkt mit dem Eingang des Licht-Steuerkreises 26 verbunden.

Zusätzlich ist vorgesehen, durch das langsam veränderliche Ausgangssignal des Signalgenerators 30 über eine Leistungsstufe 54, welche zugleich das amplitudenmodulierte Hüllkurvensignal in ein entsprechend Impulsbreitenmoduliertes Duft-Steuersignal umsetzt, ein Magnetventil 56 zu betätigen, welches über einen Druckregler 58 mit einer Preßluftflasche 60 verbunden ist. Eine an den Auslaß des Magnetventiles 56 angeschlossene Leitung taucht in ein Volumen 62 aus flüssigem Duftstoff, so daß durch Steuerung der Durchperlrate die gesteuerte Freigabe von Duftstoffen erfolgt.

Man erhält so synchron zur langsamen Veränderung der Amplitude der Lichtimpulse eine Abgabe von Duftstoffen.

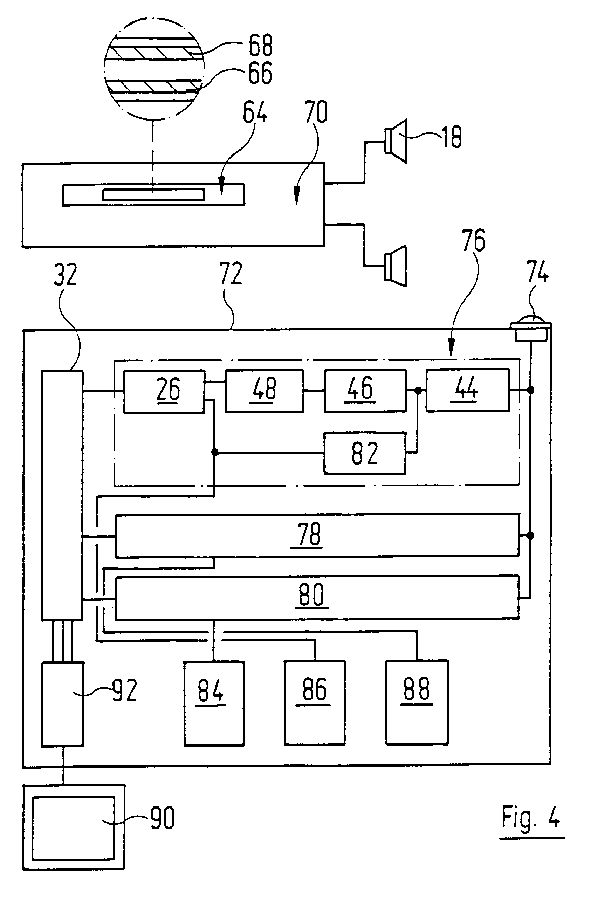

Beim abgewandelten Ausführungsbeispiel nach Figur 4 dient als Tonträger eine Musikkassette 64, bei welcher von zwei Stereo-Tonspuren 66, 68 die eine zur Musikaufzeichnung verwendet wird, während die andere zur Aufzeichnung von Steuersignalen für beispielsweise drei unterschiedliche Lichtquellen verwendet wird. Diese drei Lichtsignale liegen in deutlich getrennten hörbaren Bereichen des Spektrums, beispielsweise beim Kammerton, beim c¹ und beim f¹. Diese Signale sind amplitudenmoduliert und werden über den einen der Stereokanäle des zugehörigen, nur schematisch angedeuteten Kassettenabspielgerätes 70 wieder auf die Lautsprecher 18 gegeben.

In einem Gehäuse 72 ist ein Mikrofon 74 angebracht, welches über Steuerkanäle 76, 78, 80 einen DreifarbenLichterzeuger 32 ansteuert. Diese können jeweils aus einer Hintereinanderschaltung eines Filter 44, eines Schmidt-Triggers 46, eines Frequenzvervielfachers 48 und eines Licht-Steuerkreises 26 bestehen, wie obenstehend unter Bezugnahme auf Figur 2 im einzelnen beschrieben.

Anstelle eines separaten Hüllkurvengenerators wird bei dem Entspannungsgerät nach Figur 4 das an den Ausgängen der Filter 44 der verschiedenen Steuerkanäle 76, 78, 80 erhaltene Signal in Gleichrichterkreisen 82 gleichgerichtet, und mit der so erhaltenen langsam veränderlichen Hüllkurve der Lichtsignale, deren Amplitude beim Einspielen der Lichtsignale auf die Tonspur 68 vorgegeben wurde, werden neben den Licht-Steuerkreisen 26 zusätzlich steuerbare Duftgeneratoren 84, 86, 88 zur Abgabe unterschiedlicher Duftstoffe angesteuert.

Zusätzlich zu der Dreifarbenlichtquelle 32 wird ein Farbfernseher 90 über einen Signalumsetzer 92 mit den durch den Lichterzeuger 32 durchgeschleiften Lichtsignalen angesteuert. Der Signalumsetzer 92 setzt die Lichtsignale in geeignete Videosignale um, ähnlich zu denen, die z.B. eine Videokamera oder ein Videorecorder abgibt.

Die Verwendung des Farbfernsehgerätes 90 als steuerbare Lichtquelle hat den Vorteil, daß man eine sehr große Anzahl von Farben sehr fein im Farbton, in der Intensität und der Frequenz und ggf. auch im Muster einstellen kann. Diese Verwendung eines in den meisten Haushalten sowieso vorhandenen Farbfernsehgerätes als steuerbare Lichtquelle großer Variabilität bedingt für die Verbraucher somit auch keine zusätzlichen Kosten.

In Abwandlung des Ausführungsbeispieles nach Figur 4 kann man den Lichterzeuger 32 auch weglassen und den Farbfernseher 90 als einzige visuelle Reize abgebende Quelle verwenden.

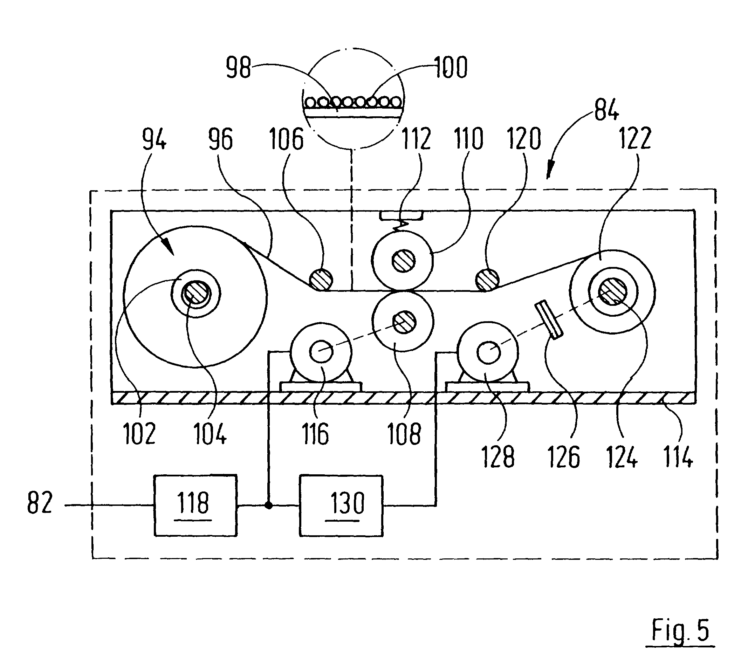

Wie aus Figur 5 ersichtlich, gehört zum Duftgenerator 84 und entsprechend zu den Duftgeneratoren 86, 88 jeweils eine Vorratsrolle 94 einer Duftträgerbahn 96, die aus einer Papierbahn 98 und hierauf angebrachten Mikrokapseln 100 besteht und auf einen Kern 102 gewickelt ist. Die Mikrokapseln können z.B. aus Gelatine bestehen und enthalten einen Duftstoff.

Die Vorratsrolle 94 ist auf einer Achse 104 frei drehbar gelagert. Die Duftträgerbahn 92 läuft über eine Leitrolle 106 in zusammenarbeitende Quetschwalzen 108, 110. Die Quetschwalze 110 ist vertikal beweglich und durch seitliche Federn 112 auf die ortsfest in einem Rahmen 114 gelagerte Quetschwalze 108 zu vorgespannt.

Die raumfeste Quetschwalze 108 wird von einem Motor 116 angetrieben, der über eine Leistungsstufe 118 das gleichgerichtete Ausgangssignal des zugeordneten Filters 44 erhält.

Über eine weitere Leitrolle 120 läuft die verbrauchte Duftträgerbahn 96 auf einen (von einer früher verwendeten Vorratsrolle verbliebenen) Kern 122, der drehfest auf einer Welle 124 sitzt. Diese wird über eine Rutschkuplung 126 von einem Motor 128 angetrieben, der durch einen Speisekreis 130 aktiviert wird. Letzter arbeitet in Abhängigkeit vom Ausgangssignal der Leistungsstufe 118, so daß der Motor 128 zusammen mit dem Motor 116, jedoch mit anderer Drehzahl angetrieben wird.

Beim Ausführungsbeispiel nach Figur 4 und 5 erhält man somit eine gesteuerte Freigabe unterschiedlicher Duftstoffe gemäß der Amplitude der auf der Tonspur 68 aufgezeichneten Lichtsteuersignale.

In Abwandlung der oben beschriebenen Ausführungsbeispiele kann man bei einem Entspannungsgerät auch gemischt steuerbare Duftquellen gemäß Figur 3 und steuerbare Duftquellen gemäß Figur 5 verwenden. Ferner kann man bei einem Entspannungsgerät gemäß Figur 3 als durch den Duftstoff 62 hindurchperlendes Trägergas Sauerstoff oder mit Sauerstoff angereicherte Luft verwenden. Es versteht sich, daß man anstelle einer Druckluftflasche auch einen kleinen Kompressor verwenden kann.

Auch kann man anstelle einer Compact Disk oder eine Musikkassette auch eine herkömmliche Schallplatte als Träger für die Tonsignale und ggf. die Lichtsignale verwenden.

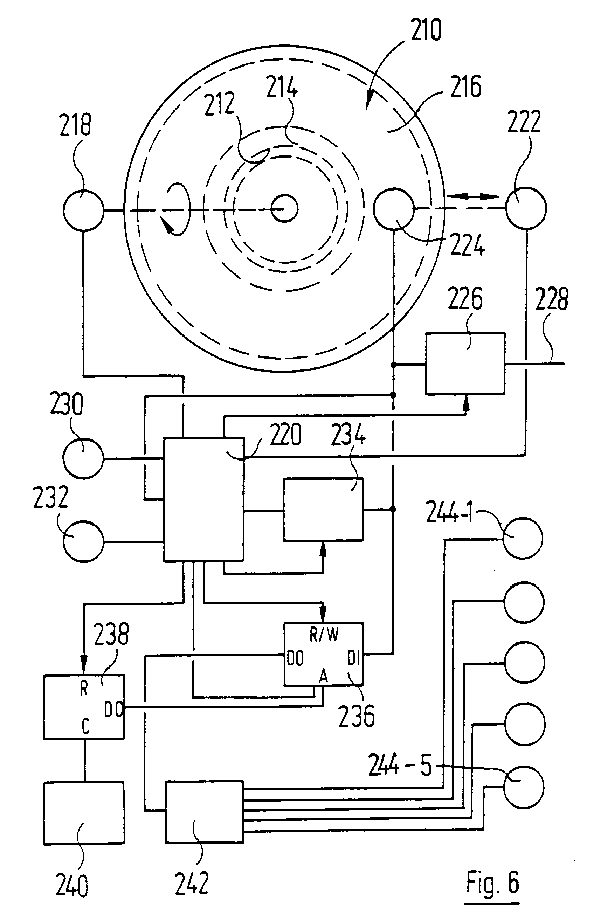

In Figur 6 ist mit 210 eine Multistimulations-Compact-Disc wiedergegeben, auf welcher drei verschiedene Aufzeichnungsbereiche abgegrenzt sind: ein Spurenbereich 212, der die Titelinformationen und Organisationsdaten enthält, ein Spurenbereich 214, der Steuersignale für Reizgeneratoren enthält, wie später noch näher beschrieben werden wird, sowie ein Spurenbereich 216, der die Tonsignale enthält.

Zum Drehen der Compact-Disc 210 ist ein Motor 218 vorgesehen, der von einer Steuereinheit 220 her erregt wird. Die Steuereinheit 220 erregt ferner einen Linearantrieb 222, durch welchen ein Lesekopf 224 in radialer Richtung über die Compact-Disc 210 verschoben wird.

Der Lesekopf 224 ist an den Eingang eines Audioteiles 226 angeschlossen, welches den für CD-Wiedergabegeräte üblichen Aufbau hat und auf einer Leitung 228 Eingangssignale für einen Verstärker bereitstellt. Die Leitung 228 ist in der Praxis eine mehradrige Leitung für den linken und rechten Stereokanal, was hier aber im einzelnen nicht ausgeführt zu werden braucht. Auch weitere in Figur 6 wiedergegebene Leitungen, insbesondere Leitungen zur Übertragung digitaler Signale sind in der Praxis mehradrige Leitungen oder Datenschienen, was aber im einzelnen hier auch nicht beschrieben zu werden braucht.

Die Steuereinheit 220 steuert das Audioteil 226 grob gesprochen so, daß sie dieses Teil totschaltet, wenn sich der Lesekopf 224 über dem Spurenbereich 212 oder dem Spurenbereich 214 befindet oder überhaupt kein Auslesen von Signalen von der Compact-Disc 210 her erfolgt.

Die Steuereinheit 220 arbeitet ihrerseits in Abhängigkeit von einem Fühler 230, der anspricht, wenn eine neue Compact-Disc in das Abspielgerät eingezogen ist. An die Steuereinheit 220 ist ferner eine Eingabeeinheit 232 angeschlossen, die zur manuellen Vorgabe des Arbeitens des Abspielgerätes dient, z.B. zur Ansteuerung eines bestimmten Titels. Ein weiterer Eingang der Steuereinheit 220 ist mit dem Ausgang des Lesekopfes 224 verbunden, damit die Steuereinheit 220 erkennen kann, wenn die vom Lesekopf 224 abgegebenen Signale Titeldaten, Organisationsdaten oder Steuersignale darstellen.

Ferner ist an den Ausgang des Lesekopfes 224 ein Organisationssignalspeicher 234 angeschlossen, der die Titel- und Organisationsdaten einspeichert, wenn der Lesekopf 224 nach dem Einlegen einer Compact-Disc 210 über den Spurenbereich 212 bewegt wird. Das Aktivieren des Organisationssignalspeichers 234 zum Einlesen dieser Daten erfolgt durch die Steuereinheit 220.

Ein Steuersignalspeicher 236 ist ebenfalls an den Lesekopf 224 angeschlossen. Er wird beim Einlesen von Daten durch die Steuereinheit 220 adressiert, die die Steuersignale für die verschiedenen im Spurenbereich 216 aufgezeichneten Stücke in getrennten Speicherfeldern ablegt.

Innerhalb des Steuersignal-Spurenbereiches 214 sind die Steuersignalfolgen für die einzelnen Musikstücken durch Trennsignale getrennt, die jeweils eine die Zuordnung zusätzlich charakterisierende Zeichenfolge umfassen. Stattdessen oder zusätzlich kann man die Steuersignalfolgen für die verschiedenen Musikstücken in fest für ein Musikstück mit bestimmter Nr. vorgegeben Sektoren und Spuren ablegen, sodaß auch über die Sektor- und Spurnummer die Zuordnung möglich ist.

Die in jedem Speicherfeld abgelegten Steuersignale enthalten die gesamte Information darüber, wie im gesamten Ablauf des zugeordneten Musikstückes eine Mehrzahl von Reizgeneratoren langsam veränderlich angesteuert werden. Die hierzu benötigten Datenmengen sind nur klein: Geht man davon aus, daß eine zeitliche Auflösung von nur 10 sec gewünscht wird, so benötigt man bei einer typischen Gesamtspieldauer einer Compact-Disc von maximal 90 min und bei einer Ansteuerung eines Reizgenerators mit 16 Bit langen Befehlen nur etwa 1 kB Speicher pro Reizgenerator. Ein preisgünstiges handelsübliches RAM mit 64 kB reicht somit für die Speicherung sämtlicher Steuerbefehle für eine große Anzahl von Reizgeneratoren aus.

Das Auslesen des Steuersignalspeichers 236 erfolgt gemäß dem Ausgangssignal eines Adressierzählers 238, der jeweils bei Beginn eines Musikstückes durch die Steuereinheit 220 zurückgesetzt wird. Das Ausgangssignal des Adressierzählers 238 bildet den niederrangigen Teil der Gesamtadresse einer Steuersignal-Speicherzelle; der höherrangige Adressteil, welcher das Speicherfeld, also das jeweilige Musikstück, identifiziert, wird auch beim Auslesen von der Steuereinheit 220 bereitgestellt.

Der Adressierzähler 238 ist an seiner Zählklemme mit einem freilaufenden Taktgeber 240 verbunden, der beispielsweise mit einer Frequenz von 0,1 Hz läuft. Diese Frequenz kann dann, wenn unter den angesteuerten Reizgeneratoren nur sehr träge arbeitende Reizgeneratoren sind, noch weiter herabgesetzt werden.

Die von dem Steuersignalspeicher 236 ausgelesenen Steuersignale gelangen auf einen Decodierer 242, der aus einem ersten Teil des Steuersignales den jeweils anzusprechenden Reizgenerator ermittelt und den Rest des Steuersignales über ein diesem speziellen Reizgenerator zugeordnete Ausgangsleitung bereitstellt. An diese Ausgangsleitungen sind verschiedene Reizgeneratoren 244-i angeschlossen, nämlich:
- eine steuerbare Lichtquelle 244-1, die in der Praxis z.B. eine Stehlampe mit steuerbarem Dimmer, aber auch eine in der Lamellenneigung elektrisch steuerbare Jalousie, ein elektrisch betätigter Vorhang oder eine Lichtorgel sein kann;
- ein taktiler Reizgenerator 244-2, der z.B. eine Servoverstellung für die Lehne und/ oder Sitzfläche und/oder Fußabstützung eines Sessels, ein in einen Sessel eingebautes Massagegerät oder dergleichen sein kann;
- ein Duftstoffgenerator 244-3, der in Abhängigkeit von den übermittelten Steuersignalen einen Duftstoff mit gewünschter Intensität abgibt;
- ein akustischer Reizgenerator 244-4, der z.B. ein anderes Abspielgerät sein kann, das in seiner Arbeitsweise langsam geändert wird (hierdurch kann man z.B. den Eindruck einer "Begegnung" mit einer anderen Klangwelt vermitteln, ähnlich wie man es z.B. in einem Konzertcafé (Störgeräusche überlagern sich der Musik) oder beim Vorbeifahren an einem Festplatz mit eingeschaltetem Autoradio erhält); oder
- ein in seiner Arbeitsweise steuerbarer visueller Reizgenerator 244-5, z.B. ein steuerbares Gebläse, welches Blätter einer Zimmerpflanze oder ein Mobile in Bewegung setzt.

Es versteht sich, daß man von jeder Kategorie auch eine Mehrzahl unterschiedlicher Reizgeneratoren vorsehen kann.

Eine weitere Abwandlung des Ausführungsbeispieles nach Figur 6 kann darin bestehen, daß der Aufzeichnungsträger nicht eine Compact-Disc für Musik- oder Sprachstücke ist sondern eine Bildplatte, wobei dann der Audioteil 226 durch ein Audio/Videoteil ersetzt ist.

Beim Ausführungsbeispiel nach Figur 6 waren die Steuersignale für alle aufgezeichneten Musikstücke gemeinsam im Spurenbereich 214 untergebracht. Die Trennung der Steuersignale für die verschiedenen Stücke erfolgt entweder durch zwischengestreute Trenn- und Identifizierungsmarken oder durch Zuordnung vorgegebener Sektoren der Steuersignalspuren zu den verschiedenen Musikstücken.

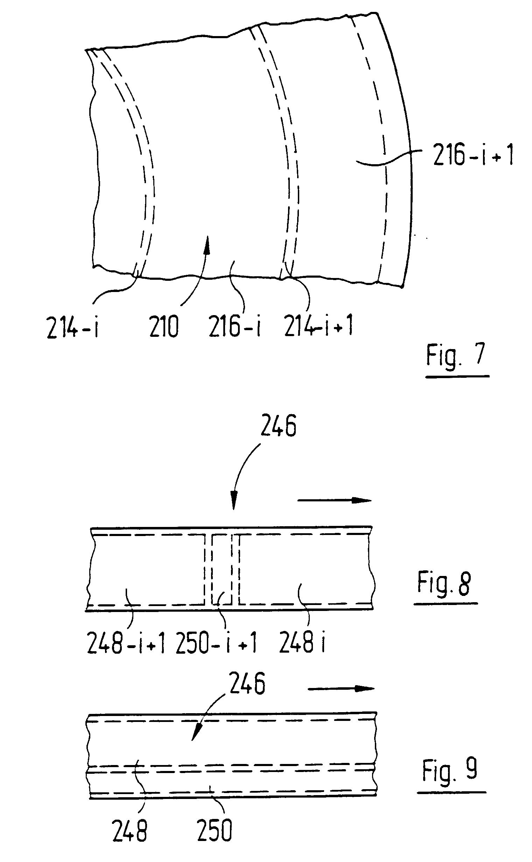

Beim abgewandelten Ausführungsbeispiel nach Figur 7 ist vor jedem einem Musikstück zugeordneten Unterbereich 216-1, 216-2 usw. des Spurenbereiches 216 der zugeordnete Unterbereich 214-1, 214-2 usw. des Steuersignal-Spurenbereiches 214 vorangestellt. Damit werden zu Beginn jedes Musikstückes automatisch die zugeordneten Steuersignale zunächst eingelesen. Die Steuereinheit 220 erkennt diese Steuersignale an entsprechenden vorgestellten Befehlscodes und leitet die Steuersignale wie beim Ausführungsbeispiel nach Figur 6 in den Steuersignalspeicher 236. Dieser braucht bei der in Figur 7 gezeigten Verschachtelung der Spurenbereiche 214 und 216 aber nur kleinere Kapazität aufweisen, bzw. man erhält bei gegebener Speichergröße eine höhere zeitliche Auflösung für die Ansteuerung der Reizgeneratoren 244. Auch das Auslesen der Steuersignale aus dem Steuersignalspeicher 236 ist vereinfacht; die Adressierung der Speicherzellen erfolgt vollständig durch den Adressierzähler 238.

Bei den Ausführungsbeispielen nach den Figuren 6 und 7 wurde davon ausgegangen, daß der Aufzeichnungsträger eine Compact-Disc ist, die Tonsignale somit auf konzentrischen Spuren aufgezeichnet sind. Es versteht sich, daß man das geschilderte Prinzip gleichermaßen auf bandförmige Aufzeichnungsträger anwenden kann, wobei das Analogon zum Ausführungsbeispiel nach Figur 6 darin besteht, daß zu Bandanfang zunächst ein Block mit den Titel- und Organisationsangaben, hierauf dann ein Block mit sämtlichen Steuersignalen und anschließend dann die Blöcke mit den verschiedenen Musikstücken kommen. Beim Abspielen des Bandes vom Anfang her werden zunächst dann die Titel und Organisationsdaten, anschließend die gesamten Steuersignale und anschließend dann die aufeinanderfolgenden Musikstücke ausgelesen. Die Ansteuerung der Reizgeneratoren kann mit derselben Elektronik und auf gleiche Weise erfolgen, wie in Figur 6 beschrieben.

Auch das Ausführungsbeispiel nach Figur 7 läßt sich analog auf bandförmiges Aufzeichnungsmaterial übertragen: Vor jedem Musikstück ist jeweils die Gruppe von Steuersignalen aufgezeichnet, die in demjenigen Zeitraum, den das Musikstück läuft, für das Ansteuern der verschiedenen Reizgeneratoren benötigt werden.

Figur 8 zeigt schematisch eine nochmals abgewandelte Art der Aufzeichnung der Steuersignale auf einem Magnetband 246. Zwischen längere Tonsignalteilblöcke 248-i eines Musikstückes sind kurze Steuersignalblöcke 250-i eingeschoben, deren Anfang und Ende jeweils durch Markierungssignale gekennzeichnet sind. Das Auslesen eines derartigen Magnetbandes kann mit einer nur geringfügig gegenüber Figur 6 abgewandelten Elektronik vorgenommen werden: Ein Ausgang der Steuereinheit 220 ist direkt mit dem Eingang des Decodierers 242 verbunden, die Schaltkreise 236 bis 240 entfallen. Die Steuereinheit 220 arbeitet so, daß sie bei Erkennen eines Steuersignalblockes 250 diesen auf den Decodierer 242 durchstellt und für diese kurze Zeit den Audioteil 226 sperrt.

In weiterer Abwandlung der Erfindung kann man gemäß Figur 9 neben einer durchgehenden Tonsignalspur 248 eine parallele durchgehende Steuersignalspur 250 vorsehen, die durch einen getrennten Lesekopf ausgelesen wird. Dessen Ausgang kann dann (ggf. unter Zwischenschaltung geeigneter Signalformkreise) direkt mit dem Eingang des Decodierers 242 verbunden werden.

Es versteht sich, daß man die Verschachtelung von Tonsignalaufzeichnung und Steuersignalaufzeichnung, wie sie für Magnetbänder anhand der Figuren 8 und 9 erläutert wurde, analog auch für umlaufende Aufzeichnungsträger vorsehen kann.

Es versteht sich ferner, daß die vorliegende Erfindung auch in Verbindung mit anderen als den schon angesprochenen Aufzeichnungsträgern verwendet werden kann, z.B. mit herkömmlichen Tonfilmen, bei denen die Steuersignale entweder in die Tonspur mit eingeblendet sind oder auf einer getrennten Steuersignalspur vorgesehen sind oder in einem Vorspann des Filmes z.B. in einigen Bildrahmen untergebracht sind, die bei stillstehendem oder langsam bewegtem Film über eine Diodenzeile oder einen Festkörperbildwandler ausgelesen werden. Weitere in Frage kommende Aufzeichnungsträger sind Minidiscs, CCDs.

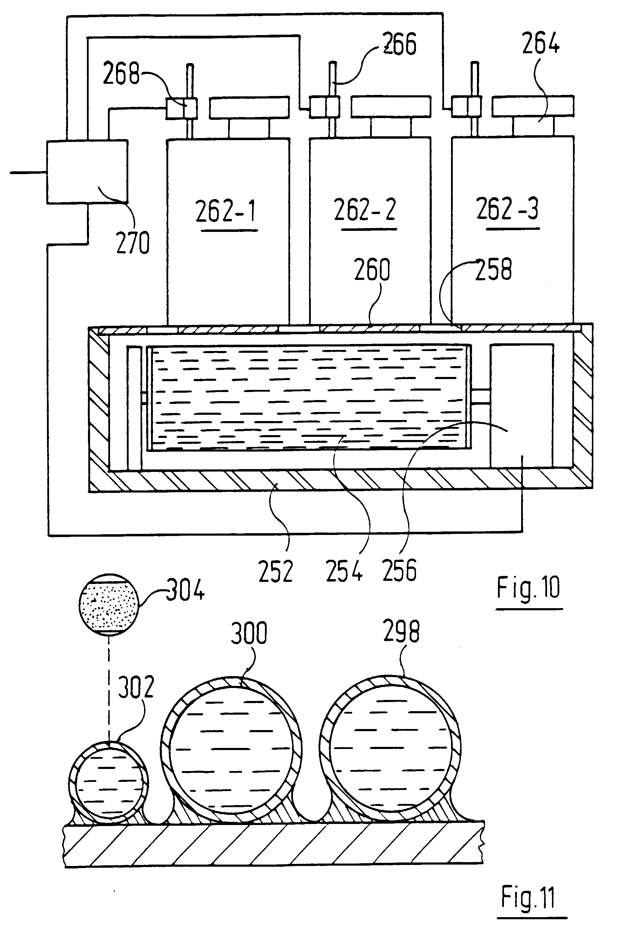

Figur 10 zeigt ein Ausführungsbeispiel für einen Duftgenerator, der drei verschiedene Duftstoffe gesteuert abgeben kann. In einem Gehäuse 252 ist ein Tangentiallüfter 254 angeordnet, der durch einen Motor 256 angetrieben wird. Die vom Tangentiallüfter 254 abgegebene Luft durchsetzt kreisbogenförmige Durchbrechungen 258 in einer Deckelplatte 260 des Gehäuses 252. Auf den zwischen den Durchbrechungen 258 verbleibenden kreisförmigen Plattenabschnitten stehen drei Duftbehälter 262-1, 262-2 und 262-3, die unterschiedliche Duftstoffe enthaltende Flüssigkeiten aufnehmen. Der über dem Flüssigkeitspegel liegende Kopfraum der Duftbehälter 262 kann mit einer Pumpe 264 unter Druck gesetzt werden, die in der Zeichnung nur schematisch angedeutet ist. In das Flüssigkeitsvolumen taucht das untere Ende eines Sprührohrs 266 ein, dessen Abgabeende durch ein Magnetventil 268 öffenbar und verschließbar ist. Die Magnetventile 268 werden zur Steuerung der Intensität des abgegebenen Duftes impulsbreitenmoduliert angesteuert. Dies besorgt ein Steuerkreis 270, der an einem Eingang das Duftsteuersignal des Decodierers 242 erhält. Sowie überhaupt ein Duftsteuersignal vorliegt, setzt der Steuerkreis 270 den Motor 256 in Gang. Je nach Art des übermittelten Duftsteuersignales legt dann der Steuerkreis 270 fest, welches bzw. welche der Magnetventile 268 aufzusteuern sind und wie das Verhältnis zwischen Offenzeit und Schließzeit einzustellen ist. Die von den Sprührohren 266 abgegebenen Duftstoffe werden durch den vom Tangentiallüfter 254 erzeugten Luftvorhang rasch in die Umgebung gefördert.

In der Praxis kann auch eine erheblich größere Zahl von Duftbehältern als drei verwendet werden, z.B. dreißig bis fünfzig Duftbehälter. Ferner können in der Praxis die Duftbehälter zu einem Block zusammengefaßt sein, wobei für die verschiedenen Magnetventile nur ein einziger gemeinsamer Stecker vorgesehen ist. Die gesamte Duftbehältereinheit kann dann als solche vertrieben und ersetzt werden.

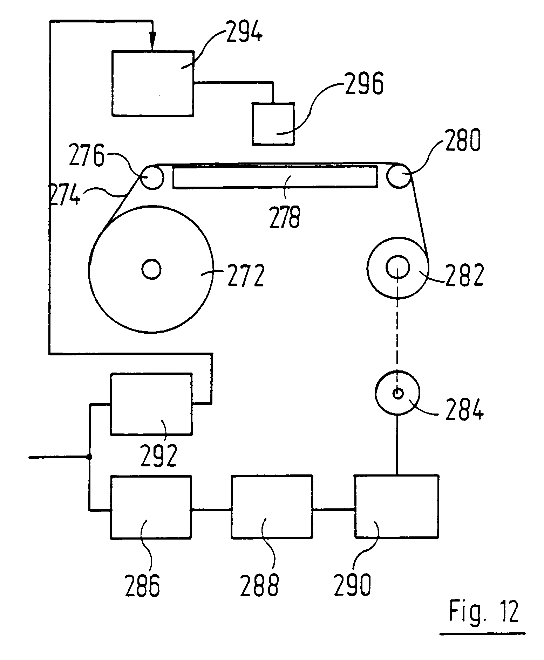

Figur 12 zeigt einen abgewandelten Duftgenerator. Von einer Vorratsrolle 272 wird ein mit Duftstoffen imprägniertes Trägerband 274 über eine Umlenkrolle 276, eine Stützplatte 278 sowie eine weitere Umlenkrolle 280 auf eine Aufwickelrolle 282 geführt. Deren Achse wird durch einen Motor 284 angetrieben, der über einen mit dem Duft-steuersignal beaufschlagten Decodierer 286, einen nachgeschalteten D/A-Wandler 288 und eine Leistungsstufe 290 gemäß der durch das Steuersignal angeforderten Intensität der Duftabgabe angetrieben wird.

An die Eingangsklemme des Duftgenerators ist ein weiterer Decodierer 292 angeschlossen, der immer dann ein Ausgangssignal bereitstellt, wenn eine von Null verschiedene Duftabgabe angefordert wird. Durch das Ausgangssignal des Decodierers 292 wird ein Speisekreis 294 für einen Ultraschallgenerator 296 angesteuert. Dieser ist über dem Trägerband 274 angeordnet, so daß die von ihm erzeugten Schallwellen auf dem Trägerband 274 vorgesehene, durch Schall aufbrechbare Mikrokapseln zerstören können, welche Duftstoffe enthalten.

Wie aus Figur 11 ersichtlich, besteht die Duftstoffimprägnierung des Trägerbandes 220 aus einer Mischung unterschiedlicher Mikrokapseln. In der Zeichnung ist bei 298 eine erste Art von Mikrokapseln wiedergegeben, die großen Durchmesser bei kleiner Wandstärke hat, also verhältnismäßig leicht durch Schallwellen aufgebrochen werden kann. Bei 300 sind Mikrokapseln wiedergegeben, die im Durchmesser den Mikrokapseln 298 entsprechen, jedoch größere Wandstärken haben und somit schwerer zu zerstören sind.

Bei 302 sind kleineren Durchmesser aufweisende Mikrokapseln gezeigt, die ein poröses Wandmaterial haben, wie in der Vergrößerung bei 304 angedeutet. Die Porosität des Wandmateriales ist jedoch gering, so daß ohne Einwirkung von außen nur geringe Duftstoffmengen durch die Wand diffundieren. Bei Wechselbelastung durch ein Schallfeld wirkt die Kapselwand dagegen ähnlich wie eine Pumpmembran, so daß man einen Durchtritt von Duftstoff hat.

Bei einem abgewandelten vereinfachten Ausführungsbeispiel kann man verschieden leicht durch Schall aufbrechbare Mikrokapseln vorsehen, die mit unterschiedlichen Duftstoffen gefüllt sind. Wählt man hierbei die mechanische Stabilität der Mikrokapseln insgesamt klein, so können diese auch durch hörbaren Schall aufgebrochen werden, so daß man ein mit mikroverkapselten Duftstoffen imprägniertes Papier auch direkt vor den Lautsprechern einer Stereoanlage aufstellen kann, wobei man dann über die Lautstärke die verschiedenen Mikrokapselarten nacheinander aufbrechen kann.

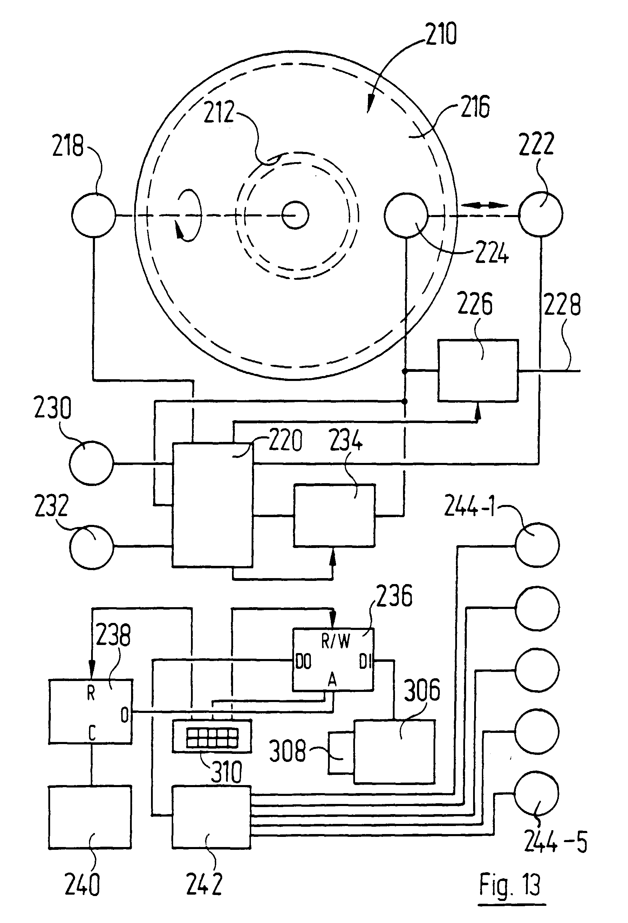

Das abgewandelte Ausführungsbeispiel nach Figur 13 entspricht in großen Teilen demjenigen nach Figur 6. Entsprechende Teile sind wieder mit denselben Bezugszeichen versehen und werden nicht nochmals im einzelnen beschrieben.

Das Einlesen der Steuersignale in den Steuersignalspeicher 236 erfolgt nun durch einen Kartenleser 306, der mit einer Steuersignalkarte 308 zusammenarbeitet. Es kann sich hierbei um eine Magnetkarte, Lochkarte oder auch eine ein ROM tragende Karte handeln. Auf der Steuersignalkarte 308 sind sämtliche Steuersignale abgelegt, die beim Ausführungsbeispiel nach Figur 6 im Steuersignal-Spurenbereich 214 der Compact-Disc 210 vorgesehen waren. Dieser Bereich ist beim Ausführungsbeispiel nach Figur 13 entfallen, das heißt, die Compact-Disc 210 ist eine handelsübliche Compact-Disc.

Die Umsteuerung des Steuersignalspeichers 236 zwischen Einlesen und Auslesen, das Rücksetzen des Zählers 238 und die Anwahl der verschiedenen Speicherfelder des Steuersignalspeichers 236 erfolgt durch ein Eingabefeld 310, welches somit die Steuereinheit 220 insoweit ersetzt, als deren Funktion über die Funktion einer normalen CD-Abspielgerät-Steuereinheit hinausgeht.

In Abwandlung des Ausführungsbeispieles nach Figur 13 kann man das Lesegerät durch ein Eingabefeld ersetzen, an welchem die Steuersignale von Hand eingegeben werden.

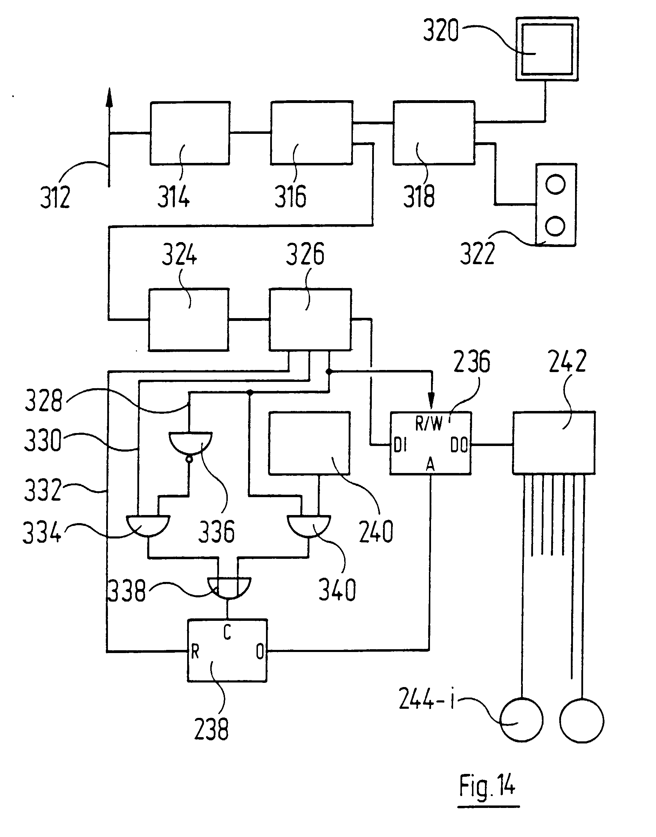

Bei dem weiter abgewandelten Ausführungsbeispiel nach Figur 14 erfolgt die Ansteuerung des Steuersignalspeichers 236 für das Auslesen ebenfalls ähnlich wie beim Ausführungsbeispiel nach Figur 6, nämlich durch einen langsam hochzählenden Zähler 238. Die Steuersignale selbst werden aber nun direkt von den Videosignalen abgeleitet, die ein Fernsehempfänger von einer Antenne 312 empfängt. An diese ist ein Eingangskreis 314 angeschlossen, der die Signale demoduliert. An dessen Ausgang ist ein Steuersignalfilter 316 angeschlossen. An dessen einem Ausgang werden die normalen Audio- und Video-ZF-Signale an ein insgesamt mit 318 bezeichnetes Audio/Videoteil weitergegeben, welches auf einen Bildschirm 320 und Lautsprecher 322 arbeitet. Am zweiten Ausgang des Steuersignalfilters 316 werden Steuersignale für Reizgeneratoren 244-i erhalten. Das Ausfiltern durch den Steuersignalfilter 316 erfolgt grob gesprochen so, daß dieser Signalfolgen, welche durch ein spezielles Trennsignal eingeleitet und beendet werden, aus dem Strom der Signale herausnimmt. Diese herausgefilterten Signale werden in einem Analog/Digital-Wandler 324 in binäre Signale umgesetzt, und diese kommen auf eine Steuereinheit 326. Diese stellt anhand der Trennsignale zunächst fest, wann eine Steuersignalübertragung beginnt und endet. Ein entsprechendes Steuersignal wird auf einer Leitung 328 bereitgestellt. Durch dieses Signal wird das Umschalten des Steuersignalspeichers 236 zwischen Einlesen (W) und Auslesen (R) bewerkstelligt. Auf einer weiteren Leitung 330 stellt die Steuereinheit 326 immer dann ein Signal bereit, wenn ein neues, z.B. 1 oder 2 Byte umfassendes Steuersignal erhalten wurde. Auf einer weiteren Leitung 332 stellt die Steuereinheit 326 jeweils zu Beginn eines Steuersignalpaketes bzw. am Ende eines Steuersignalpaketes ein Signal bereit. Durch dieses Signal wird der Zähler 238 zurückgesetzt.

Ein UND-Glied 334 erhält die auf der Leitung 330 stehenden Taktsignale. Sein zweiter Eingang ist über einen Inverter 336 mit dem Aktivierungssignal der Leitung 328 beaufschlagt. Der Ausgang des UND-Gliedes 334 ist mit einem ersten Eingang eines ODER-Gliedes 338 verbunden, dessen Ausgang mit der Zähklemme C des Zählers 238 verbunden ist. Ein zweiter Eingang des ODER-Gliedes 338 erhält über ein UND-Glied 340 die Ausgangssignale des freilaufenden Taktgebers 240. Der zweite Eingang des UND-Gliedes 340 wird mit dem Signal auf der Leitung 328 beaufschlagt.

Damit zählt der Zähler 238 beim Einlesen des Steuersignalspeichers 236 immer um eins hoch, wenn ein neues Steuersignal erhalten wurde. Beim Auslesen des Steuersignalspeichers 236 zählt der Zähler 238 gemäß den Impulsen des Taktgebers 240 hoch.

Die Dateneingangsklemme DI des Steuersignalspeichers 236 ist mit einer Ausgangsklemme der Steuereinheit 326 verbunden, an welcher die digitalisierten Steuersignale abgegeben werden. Eine Datenausgangsklemme DO des Steuersignalspeichers 236 ist wieder mit dem Decodierer 242 verbunden, welcher die verschiedenen Duftgeneratoren 244-i auswählt und diesen die entsprechenden Steuersignale zuleitet.

Das oben beschriebene Fernsehgerät arbeitet folgendermaßen:

Die Steuersignale für die Reizgeneratoren 244-i werden vorzugsweise zu einem Block zusammengefaßt, der dem Inhalt des Steuersignal-Spurenblockes 214 beim Ausführungsbeispiel von Figur 6 entspricht. Dieser Block wird zu Beginn einer Sendung in einer Frequenz lücke ausgestrahlt und vom Steuersignalfilter 316 abgetrennt. Die Steuersignale werden dann im Steuersignalspeicher 236 abgelegt, wie oben beschrieben. Nach Übertragung des Steuersignalblockes wird der Zähler 238 durch die Steuereinheit 326 wieder zurückgesetzt, und nun wird der Steuersignalspeicher 236 langsam ausgelesen, wie für das Ausführungsbeispiel nach Figur 6 im einzelnen erläutert. Damit werden die Reizgeneratoren an den gewünschten Stellen der folgenden Sendung mit der jeweils gewünschten Intensität angeschaltet.

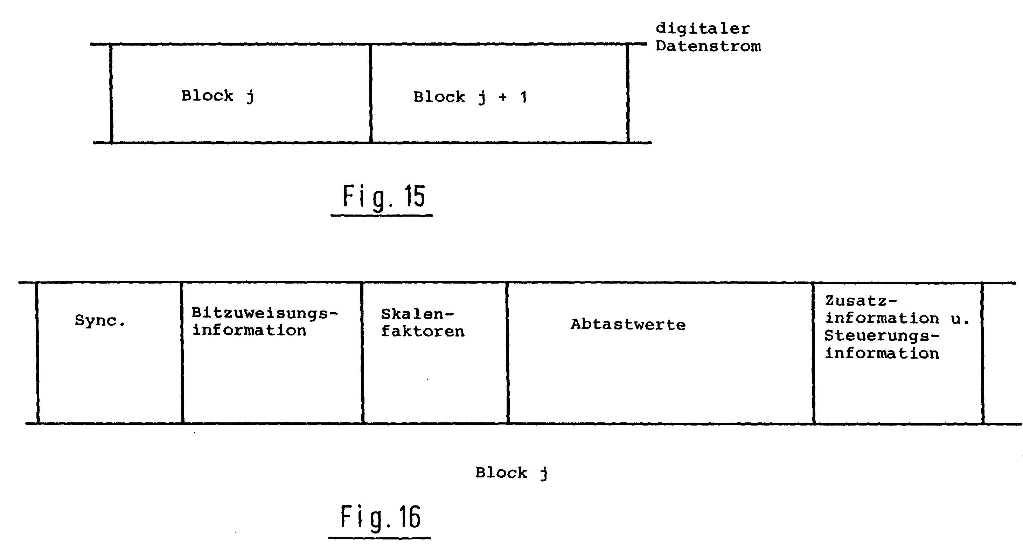

Der in Figur 15 dargestellte Datenstrom ist auf einem digitalen Tonträger, z.B. Platte oder Magnetbandcassette, oder auf der Begleittonspur eines Bildträgers, z.B. Videoplatte oder Videobandcassette, aufgezeichnet. Der Datenstrom besteht aus seriellen Datenblöcken (= festgelegte Zeitintervalle), von denen die Datenblöcke j und j+1 in Figur 15 angedeutet sind, um die serielle Blockfolge zu veranschaulichen. Jeder Datenblock weist die in Figur 16 angedeutete Struktur auf, bei welcher der Anfang jedes Datenblocks durch die als "Header" (Kopf) vorgesehene Synchronisierungsinformation "Sync" identifizierbar ist. Im Falle von nichtkomprimierten, 16-Bit linear-codierten Tonsignalen (z.B. bei CDs oder DAT-Kassetten) schließt sich an den Header "Sync" unmittelbar eine Folge von Abtastwerten des digitalisierten Tonsignals an. Bei den in Figur 16 veranschaulichten datenkomprimierten Tonsignalen (z.B. MiniDiscs oder DCC-Kassetten) sind zwischen dem Header "Sync" und der Folge der Abtastwerte ein Abschnitt "Bit-Zuweisungsinformationen" und ein Abschnitt "Skalenfaktoren" eingefügt.

Im Anschluß an den Abschnitt "Abtastwerte" ist in jedem Datenblock ein Abschnitt für Zusatzinformationen reserviert. Falls keine Zusatzinformation vorgesehen ist, werden in den betreffenden Abschnitt des Datenblocks logische Nullen eingefügt. Bei bekannten digitalen Tonträgern ist als Zusatzinformation z.B. die Numerierung und/oder die Titel der einzelnen Aufzeichnungen vorgesehen.

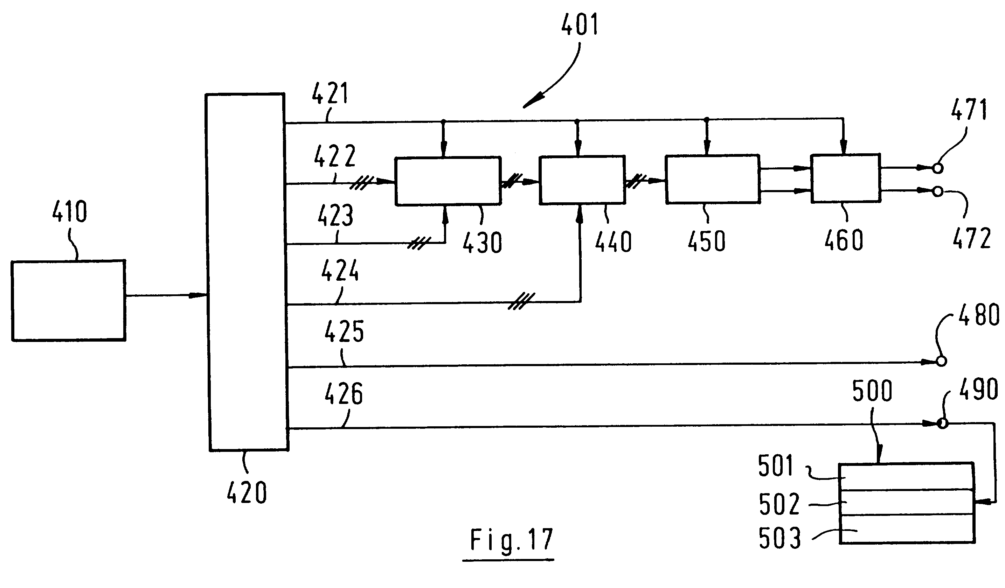

Erfindungsgemäß ist in dem für Zusatzinformationen reservierten Abschnitt jedes Datenblocks zusätzlich zu oder anstelle von der herkömmlichen Zusatzinformation eine spezielle Steuerungsinformation für einen optischen, akustischen und/oder duftstoffproduzierenden Reizgenerator 500 (Figur 17) eingefügt. Diese Steuerungsinformation unterscheidet sich von der sonstigen Zusatzinformation durch entsprechend unterschiedliche Codierung oder durch geeignete Identifikationsbits. Die Codierung der Steuerungsinformation ist an die Codierung der Abtastwerte (Teilband- oder Transformationscodierung) angepaßt, wodurch in vorteilhafter Weise ein Multiplex der Daten des digitalisierten Tonsignals (einschließlich Zusatzinformation) und der Daten der erfindungsgemäßen Steuerungsinformation ermöglicht wird.

Die Decodierung von Datenblöcken gemäß Figur 16 in einem Abspielgerät ist anhand des Blockschaltbildes nach Figur 17 erläutert. Das Platten- oder Bandlaufwerk 410 des Abspielgerätes liest den Datenstrom gemäß Figur 15 aus und führt ihn dem Demultiplexer 420 des Decoders 401 zu. Aus den seriell ankommenden Datenblöcken des Datenstroms selektiert der Demultiplexer 420 die in jedem Datenblock enthaltenen Abschnitte und stellt die zugehörigen Datenbits an gesonderten Ausgängen 421 bis 426 zur Verfügung. Im gezeigten Beispielsfall von Figur 17 werden
- dem Ausgang 421 die Synchronisierungsinformation "Sync",
- dem Ausgang 422 die Abtastwerte,
- dem Ausgang 423 die Bit-Zuweisungsinformation,
- dem Ausgang 424 die Skalenfaktoren,
- dem Ausgang 425 die herkömmliche Zusatzinformation, und
- dem Ausgang 426 die erfindungsgemäße Steuerungsinformation

zugeführt. Die Ausgänge 422, 423, 424 und 425 sind im Falle einer Aufspaltung des digitalisierten Tonsignals in 16 Teilbänder (Teilbandcodierung) als jeweils 16-faches Leitungsbündel mit einer Leitung je Teilband ausgeführt. Für jedes Teilband ist eine gesonderte Serienschaltung aus den Blöcken 430, 440 und 450 (Figur 17) vorgesehen, wie durch jeweils drei Schrägstriche in den Zuführungsund Verbindungsleitungen zu bzw. zwischen den Blöcken 430, 440 und 450 angedeutet ist. Die folgende Betrachtung der Funktionsweise der Blöcke 430, 440 und 450 gilt daher für jedes Teilband in identischer Weise.

Die Folge der Abtastwerte am Ausgang 422 wird einer Bitzuweisungsstufe 430 zugeführt, deren Steuereingang 431 mit dem Ausgang 423 verbunden ist. Entsprechend der BitZuweisungsinformation am Steuereingang 431 werden die irrelevanz-reduzierten Abtastwerte de-quantisiert und in der nachfolgenden Normierungsstufe 440 mit Hilfe der dort am Steuereingang 441 anliegenden Skalenfaktoren gepegelt. Nach erfolgter Normierung werden sämtliche 16 Teilbandsignale dem gemeinsamen inversen Filter 450 zugeführt und dort zu einem breitbandigen, stereophonen Digitalsignal vereinigt. Dieses stereophone Digitalsignal wird in dem Digital/Analog-Wandler 460 in ein stereophones Analogsignal umgewandelt und steht mit seinem Linksanteil L am Ausgang 471 sowie mit seinem Rechtsanteil R am Ausgang 472 des Decoders 401 zur Verfügung.

Die am Ausgang 425 des Demultiplexers 420 anliegende Zusatzinformation wird einem Ausgang 480 des Decoders 401 zugeführt, während die am Ausgang 426 des Demultiplexers anliegende, erfindungsgemäße Steuerungsinformaion an einem Ausgang 490 des Decoders 410 zur Verfügung gestellt wird. An den Ausgang 490 läßt sich ein Reizgenerator 500 anschließen, welcher schematisch in einem optischen Teil 501, einen akustischen Teil 502 und einen duftstoffproduzierenden Teil 503 unterteilt ist. In der Praxis handelt es sich bei den Teilen 501 bis 503 des Reizgenerators um gesonderte Geräte, deren Steuereingänge jeweils über eine Datenbusverbindung mit dem Ausgang 490 des Decoders 401 verbunden sind. Die beispielhafte Ausbildung der Teile 501 bis 503 des Reizgenerators 500 wurde vorstehend bereits erläutert. Anstelle von Mikrokapseln mit Duftstoffen, die durch Ultraschallsignale freigesetzt werden, ist es alternativ auch möglich, Duftstoff-Flaschen zu verwenden, deren Verschluß über ein Magnetventil von der digitalen Steuerungsinformation am Ausgang 490 des Decoders 401 gesteuert wird. Für den optischen Teil 501 des Reizgenerators 500 kann beispielsweise eine sogenannte "Lichtorgel" mit verschiedenfarbigen Lampen verwendet werden, die gegebenenfalls motorisch nach vorgegebenen Programmen verschwenkbar sind, um in einem Raum sich ständig verändernde Beleuchtungseffekte zu erzielen. Sowohl die Aufeinanderfolge der Farben als auch die Bewegung der einzelnen Farblampen lassen sich mit Hilfe der Steuerungsinformation am Ausgang 490 des Decoders 401 steuern, um beispielsweise passend zur Musik die Beleuchtung eines Raumes rhythmisch zu verändern.

Im Falle eines Tongenerators als Teil 502 des Reizgenerators 500 können anstelle oder zusätzlich zu der Steuerungsinformation am Ausgang 490 auch die Skalenfaktoren zur Steuerung verwendet werden, welche - wie schon erwähnt-die Einhüllende bzw. Hüllkurve des digitalisierten Tonsignals darstellen. Durch direkte Wiedergabe dieser Einhüllenden oder durch Modulation von Tonschwingungen mit dieser Einhüllenden entstehen spezielle Klangeffekte, die bei Wiedergabe zusätzlich zu dem aufgezeichneten Tonsignal beruhigende oder stimulierende Wirkungen auf den Hörer auslösen können.

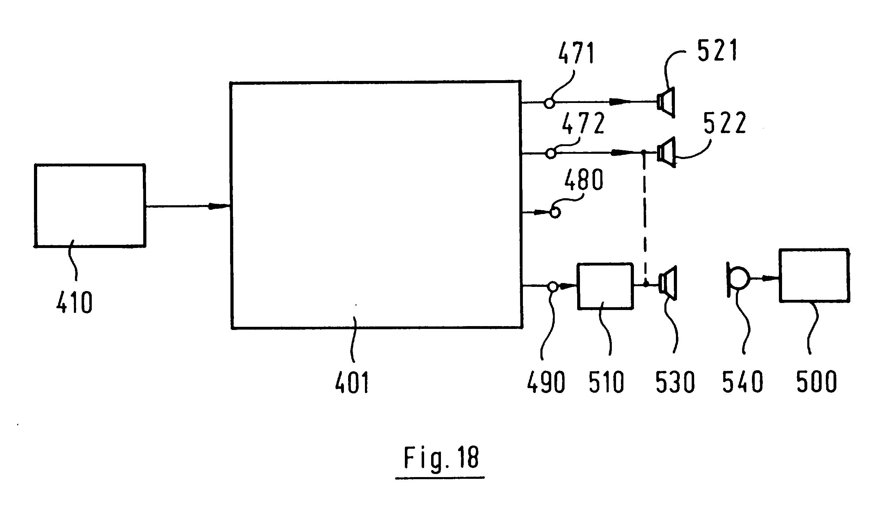

Anstelle einer galvanischen Verbindung zwischen dem Reizgenerator 500 und der Steuerungsinformation am Ausgang 490 ist, wie anhand von Figur 18 dargestellt ist, auch eine akustische Kopplung im Sinne einer drahtlosen Fernsteuerung des Reizgenerators 500 möglich. Hierzu wird die Steuerungsinformation am Ausgang 490 einem Schallgenerator 510 zugeführt, welcher ein Schallsignal im hörbaren oder im unhörbaren (Ultraschall-) Bereich erzeugt. Dieses Schallsignal wird entweder über einen gesonderten elektroakustischen Wandler 530 oder - im Falle eines hörbaren Schallsignals - über einen (521) der Lautsprecher 521, 522 des Abspielgerätes abgestrahlt, welche über nicht dargestellte Verstärker mit den Ausgängen 471 bzw. 472 für das analoge stereophone Tonsignal verbunden sind. Der Reizgenerator 500 ist in diesem Beispielsfall mit einem Mikrophon 540 versehen, welches das hörbare oder unhörbare Schallsignal detektiert und als Steuerungsinformation dem Steuereingang des Reizgenerators 500 zuführt. Ein hörbares Schallsignal kann so gewählt werden, daß es - ähnlich wie bei der vorstehend erwähnten Ausnutzung der Einhüllenden des Tonsignals - eine beruhigende oder stimulierende Wirkung auf den Hörer auslösen kann.

## Patentansprüche

1. Gerät zum Erzeugen von Tönen und/oder Bildern, mit einer Nutzsignalquelle (210, 224; 312, 314) für Ton- und/oder Bildsignale und mit einer hiermit verbundenen Ausgabeeinheit (226, 228; 318 bis 322) für Töne und/oder Bilder, mit einer Steuersignalquelle (210, 224, 236 bis 240; 306, 308, 236 bis 240; 316, 324, 326, 236 bis 240) und mindestens einem durch diese gesteuerten Reizgenerator (244), wobei die Reizgenerator-Steuersignale verglichen mit den Nutzsignalen kleine Änderungsgeschwindigkeit haben dadurch gekennzeichnet, daß die Steuersignalquelle umfaßt: einen Steuersignalgenerator (214, 224; 306, 308; 312 bis 316) sowie einen an diesen angeschlossenen Steuersignalspeicher (236) sowie eine Steuereinheit (220; 310; 326), durch welche der Steuersignalspeicher (236) für das Ein- und Auslesen von Steuersignalen aktivierbar ist; und daß der Steuersignalspeicher (236) ein digitaler Speicher ist und seine Speicherzellen beim Auslesen durch einen Adressierzähler (238) adressiert werden, dessen Zählklemme (C) mit dem Ausgang eines langsamen freilaufenden Taktgebers (240) verbunden ist, während die Adressierung des Steuersignalspeichers (236) beim Einlesen der Steuersignale durch die Steuereinheit (220; 326) in Abhängigkeit von den einlaufenden Steuersignalen erfolgt.

2. Gerät nach Anspruch 1, dadurch gekennzeichnet, daß die Steuersignalquelle (210, 224; 236 bis 240; 306, 308, 236 bis 240; 312 bis 316, 236 bis 240) über einen Steuersignaldecodierer (242) eine Mehrzahl von Reizgeneratoren (244-i) ansteuert.

3. Gerät nach Anspruch 1 oder 2, wobei die Nutzsignalquelle ein Rundfunk/Fernseh-Empfangsteil (312, 314, 318) umfaßt, dadurch gekennzeichnet, daß die Steuersignalquelle ein Steuersignalfilter (316) aufweist, welches aus dem Strom von einem Eingangskreis (314) des Rundfunk/Fernseh-Empfangsteiles (314, 318) abgegebener Signale frequenzmäßig oder zeitlich oder durch Trennsignale abgesetzte Steuersignale herausfiltert.

4. Gerät nach Anspruch 1 oder 2, wobei die Nutzsignalquelle einen Aufzeichnungsträger für Ton und/oder Bild umfaßt, bei welchem auf einem Trägermedium (210; 246) Ton- und/oder Bildsignale enthaltende Spuren (216; 248) angebracht sind, dadurch gekennzeichnet, daß auf dem Trägermedium (210; 246) zusätzlich Steuersignale (214; 250) zur Ansteuerung des Reizgenerators vorgesehen sind.

5. Gerät nach einem der Ansprüche 1 bis 4 für einen Aufzeichnungsträger, bei welchem die jeweils einer der Ton- und/oder Bildsignal-Steuergruppen (216-i) zugeordneten Steuersignalspurengruppen zusammenhängend in einem Steuersignal-Spurenbereich (214) aufgezeichnet sind, wobei die Steuersignal-Spurengruppen für verschiedene Stücke durch Trennsignale getrennt und vorzugsweise auch identifiziert sind, dadurch gekennzeichnet, daß eine Teiladressierung des Steuersignalspeichers (236) beim Auslesen von der Steuereinheit (230; 310) gemäß der Nummer des jeweils abzuspielenden Stückes erfolgt.

6. Gerät nach einem der Ansprüche 1 bis 5, dadurch gekennzeichnet, daß der Steuersignalgenerator einen Steuersignalträger (308) und ein zugeordnetes Lesegerät (306) oder eine Steuersignaleingabeeinheit aufweist.

7. Gerät zum Erzeugen nach einem der Ansprüche 1 bis 6, dadurch gekennzeichnet, daß die Steuersignalquelle Phasenkopplungsmittel (10, 22, 24; 42, 48; 52, 48) aufweist, die mit dem Ton- und/oder Bild-Nutzsignal beaufschlagt sind und an ihrem Ausgang das zum Ton- und/oder Bild- Nutzsignal vorgegebene Phasenlage aufweisende Reizgenerator-Steuersignal bereitstellen.

8. Gerät nach Anspruch 7, dadurch gekennzeichnet, daß die Phasenkopplungsmittel einen mit dem Tonsignal beaufschlagten, z.B. niedere Frequenzen durchlassenden, Filterkreis (44) aufweisen, dessen Eingang mit dem Ton- und/oder Bild-Nutzsignal beaufschlagt ist und dessen Ausgang das Reizgenerator-Steuersignal bereitstellt.

9. Gerät nach Anspruch 7, dadurch gekennzeichnet, daß die Nutzsignalquelle ein Musikinstrument ist, welches einen den Takt einer Begleitmusik vorgebenden Frequenzteiler (22, 24) aufweist, dessen Eingang mit einem freilaufenden Taktgeber (10) verbunden ist und dessen Ausgang mit einem Klanggenerator (34) verbunden ist, und daß die Phasenkopplungsmittel durch den Frequenzteiler (22, 24) gebildet sind, welcher an einem Zwischenausgang das Reizgenerator-Steuersignal bereitstellt.

10. Gerät nach einem der Ansprüche 1 bis 9, dadurch gekennzeichnet, daß die Steuersignalquelle einen einstellbaren Frequenzteiler (22, 24) oder Frequenzvervielfacher (48) aufweist, dessen Eingang mit dem Ton- und/oder Bild-Nutzsignal beaufschlagt ist und dessen Ausgang das Reizgenerator-Steuersignal bereitstellt.

11. Gerät nach einem der Ansprüche 1 bis 10, dadurch gekennzeichnet, daß die Steuersignalquelle einen Hüllkurvengenerator (30) aufweist, dessen Ausgangssignal auf einen Steuerkreis (26) gegeben wird, welcher an einem weiteren Eingang mit dem Reizgenerator-Steuersignal beaufschlagt ist und letzteres gemäß dem vom Hüllkurvengenerator (30) erzeugten Ausgangssignal Amplituden moduliert.

12. Gerät nach einem der Ansprüche 1 bis 11, dadurch gekennzeichnet, daß mindestens einer der steuerbaren Reizgeneratoren ein steuerbarer Duftgenerator (56-62; 84; 244-i) ist.

13. Gerät nach Anspruch 12, dadurch gekennzeichnet, daß mindestens einer der steuerbaren Duftgeneratoren aufweist: einen steuerbaren Trägergasgenerator (56-60) sowie ein Duftstoffvolumen (62), durch welches das Trägergas hindurchperlt.

14. Gerät nach Anspruch 13, dadurch gekennzeichnet, daß das Trägergas Sauerstoff oder im Sauerstoffgehalt angereicherte Luft ist.

15. Gerät nach einem der Ansprüche 12 bis 14, dadurch gekennzeichnet, daß mindestens einer der steuerbaren Duftgeneratoren aufweist: einen Duftträger (96), bestehend aus einer Unterlage (98) und mindestens einem auf dieser aufgebrachten mikroverkapselten Duftstoff sowie eine steuerbare Kapselbrecheinrichtung (104 bis 112), die nacheinander mit unterschiedlichen Bereichen des Duftträgers (96) zusammenarbeitet.

16. Gerät nach Anspruch 15, dadurch gekennzeichnet, daß der Duftträger (96) bahnförmig ist und von der Quetschwalzen (104, 106) aufweisenden Kapselbrecheinrichtung von einer Vorratsrolle (94) abgezogen wird.

17. Gerät nach einem der Ansprüche 12 bis 16, dadurch gekennzeichnet, mindstens einer der steuerbaren Duftgeneratoren (244-i) aufweist: einen unter Druck setzbaren (264) Behälter (262-i), der mit mindestens einen Duftstoff enthaltender Flüssigkeit gefüllt ist, ein in die Duftstoffe enthaltende Flüssigkeit eintauchendes Sprührohr (266) sowie ein in das Sprührohr (266) geschaltetes steuerbares Abgabeventil (268).

18. Gerät nach Anspruch 17, dadurch gekennzeichnet, daß mehrere Behälter (262-i) für Duftstoffe enthaltende Flüssigkeiten zu einem Behälterblock verbunden sind.

19. Gerät nach Anspruch 17 oder 18, dadurch gekennzeichnet, daß in der Nachbarschaft der Sprührohre (266) ein Gebläse (254) angeordnet ist.

20. Gerät nach einem der Ansprüche 12 bis 19, dadurch gekennzeichnet, daß mindestens einer der Duftgeneratoren (244-i) aufweist: ein Substrat (274) sowie auf diesem angeordnete Mikrokapseln (298 bis 302), welche einen Duftstoff enthalten, wobei das Wandmaterial der Mikrokapseln entweder spröde und durch Schall brechbar ist oder nachgiebig und porös ist, sowie einen Schall gegen die Mikrokapseln richtenden Schallgenerator (296).

21. Gerät nach Anspruch 20, dadurch gekennzeichnet, daß die Mikrokapseln (298 bis 302) ein Gemisch aus unterschiedlichen Kapseln ist, die bei unterschiedlicher Frequenz und/oder unterschiedlicher Schallintensität ansprechen, z.B. Kapseln mit unterschiedlichem Wandmaterial, Kapseln mit unterschiedlichem Durchmesser, Kapseln mit unterschiedlicher Wandstärke.

22. Gerät nach Anspruch 20 oder 21, dadurch gekennzeichnet, daß der Schallgenerator ein Lautsprecher einer Ton- wiedergabeeinrichtung ist.

23. Gerät nach Anspruch 20 oder 21, dadurch gekennzeichnet, daß der Schallgenerator ein Ultraschallgenerator (296) ist, der in unmittelbarer Nähe des die Mikrokapseln (298 bis 302) tragenden Substrates (274) angeordnet ist.

24. Gerät nach Anspruch 22 oder 23, gekennzeichnet durch eine hinter dem Substrat (274) angeordnete harte Wand (278).

25. Gerät nach einem der Ansprüche 20 bis 24, dadurch gekennzeichnet, daß das Substrat (274) bandförmig ist und durch einen Antrieb (284) gemäß dem Duftsteuersignal am Schallgenerator (296) vorbei bewegt wird.

26. Gerät nach einem der Ansprüche 1 bis 25, dadurch gekennzeichnet, daß durch das Reizgenerator-Steuersignal zugleich ein Klanggenerator (34) gesteuert wird, dessen Ausgangssignal dem Ton-Nutzsignal zugemischt wird.

27. Gerät nach einem der Ansprüche 1 bis 26 dadurch gekennzeichnet, daß mindestens einer (32) der steuerbaren Reizgeneratoren mit einem Koppelmikrofon (74) bzw. einem Lichtdetektor zusammenarbeitet, welches bzw. welcher auf von einem Lautsprecher (18) bzw. einem Bilderzeuger mit abgestrahlte, den Steuersignalen zugeordnete Signale anspricht.

28. Gerät nach Anspruch 27, dadurch gekennzeichnet, daß eine Reizgenerator-Betriebseinheit, das Koppelmikrofon (74) und vorzugsweise auch der Reizgenerator (32) selbst in einem gemeinsamen Gehäuse (72) untergebracht sind.

29. Gerät nach einem der Ansprüche 1 bis 28, dadurch gekennzeichnet, daß einer (32) der Reizgeneratoren ein Farbfernsehgerät (90) ist, das über einen Signalumsetzer (92) mit den Steuersignalen beaufschlagt ist, welcher die Steuersignale in Videosignale umsetzt.

## Claims

1. Appliance for generating sounds and/or images, with a useful signal source (210, 224; 312, 314) for sound and/or image signals and with an output unit (226, 228; 318 to 322) connected therewith for sound and/or images, with a control signal source (210, 224, 236 to 240; 306, 308, 236 to 240; 316, 324, 326, 236 to 240) and at least one stimulus generator (244) controlled by the latter, wherein the stimulus generator control signals have a small rate of variation compared with the useful signals, characterised in that the control signal source comprises: a control signal generator (214, 224; 306, 308; 312 to 316) together with a control signal memory (236) connected to the latter and a control unit (220; 310; 326) by means of which the control signal memory (236) is activatable for the reading in and out of control signals ; and that the control signal memory (236) is a digital memory and its memory cells are addressed during the reading out by an address counter (238) whose counting terminal (C) is connected to the output of a slow free-running clock (240), while the addressing of the control signal memory (236) during the reading in of the control signals by the control unit (220; 326) takes place as a function of the incoming control signals.

2. Appliance according to claim 1, characterised in that the control signal source (210, 224, 236 to 240; 306, 308, 236 to 240; 312 to 316, 236 to 240) controls a plurality of stimulus generators (244-i) by means of a control signal decoder (242).

3. Appliance according to claim 1 or 2, in which the useful signal source incorporates a radio/television reception part (312, 314, 318), characterised in that the control signal source comprises a control signal filter (316) which filters out of the flow of signals emitted by an input circuit (314) of the radio/television reception part control signals contrasted frequency-wise or time-wise or by means of separating signals.

4. Appliance according to claim 1 or 2, in which the useful signal source incorporates a record support for sound and/or image, in which tracks (216; 248) containing sound and/or image signals are accommodated on a support medium (210; 246), characterised in that control signals (214; 250) for driving the stimulus generator are provided additionally on the support medium (210; 246).

5. Appliance according to one of claims 1 to 4 for a record support, in which the control signal track groups assigned to one or the other of the sound and/or image signal control groups (216-i) are recorded coherently in a control signal track area (214), wherein the control signal track groups are separated for various pieces and preferably also identified by means of separating signals, characterised in that a partial addressing of the control signal memory (236) during the reading out from the control unit (230; 310) takes place according to the number of the particular piece to be played back.

6. Appliance according to one of claims 1 to 5, characterised in that the control signal generator comprises a control signal support (308) and an associated reader (306) or a control signal input unit.

7. Appliance for generating according to one of claims 1 to 6, characterised in that the control signal source comprises phase coupling means (10, 22, 24; 42, 48; 52, 48) which are impressed with the useful sound and/or image signal and produce at their outlet the stimulus generator control signal having the prescribed phase position for the useful sound and/or image signal.

8. Appliance according to claim 7, characterised in that the phase coupling means comprise a filter circuit (44) impressed with the sound signal, e.g. letting through lower frequencies, the input of which filter circuit (44) is impressed with the useful sound and/or image signal and the output-of which filter circuit (44) produces the stimulus generator control signal.

9. Appliance according to claim 7, characterised in that the useful signal source is a musical instrument which comprises a frequency divider (22, 24) pre-determining the rhythm of an accompanying music, the input of which frequency divider (22, 24) is connected to a free-running clock (10) and the output of which is connected to a sound generator (34), and that the phase coupling means are formed by the frequency divider (22, 24), which produces the stimulus generator control signal at an intermediate output.

10. Appliance according to one of claims 1 to 9, characterised in that the control signal source comprises an adjustable frequency divider (22, 24) or frequency multiplier (48), whose input is impressed with the useful sound and/or image useful signal and whose output produces the stimulus generator control signal.

11. Appliance according to one of claims 1 to 10, characterised in that the control signal source comprises an envelope curve generator (30) whose output signal is passed to a control circuit (26) which is impressed at a further input with the stimulus generator control signal and the latter modulates amplitudes in accordance with the output signal generated by the envelope curve generator (30).

12. Appliance according to one of claims 1 to 11, characterised in that at least one of the controllable stimulus generators is a controllable fragrance generator (56-62; 84; 244-i).

13. Appliance according to claim 12, characterised in that at least one of the controllable fragrance generators comprises: a controllable carrier gas generator (56-60) as well as a fragrance volume (62) through which the carrier gas bubbles.

14. Appliance according to claim 13, characterised in that the carrier gas is oxygen or air enriched in oxygen content.

15. Appliance according to one of claims 12 to 14, characterised in that at least one of the controllable fragrance generators comprises: a fragrance support (96), consisting of a base (98) and at least one micro-encapsulated fragrance placed on the latter together with a controllable capsule-breaking device (104 to 112) which interacts successively with different areas of the fragrance support (96).

16. Appliance according to claim 15, characterised in that the fragrance support (96) is band-shaped and is withdrawn from a delivery spool (94) by the capsule-breaking device comprising squeezing rolls (104, 106).

17. Appliance according to one of claims 12 to 16, characterised in that at least one of the controllable fragrance generators (244-i) comprises: a vessel (262-i) placeable under pressure (264) which is filled with liquid containing at least one fragrance, a spray pipe (266) dipping into the liquid containing fragrances, and a controllable outlet valve (268) fitted into the spray pipe (266).

18. Appliance according to claim 17, characterised in that several vessels (262-i) for liquids containing fragrances are combined into a block of vessels.

19. Appliance according to claim 17 or 18, characterised in that a blower (254) is disposed in the vicinity of the spray pipes (266).

20. Appliance according to one of claims 12 to 19, characterised in that at least one of the fragrance generators (244-i) comprises: a substrate (274) and, arranged on the latter, microcapsules (298 to 302) which contain a fragrance, wherein the wall material of the microcapsules is either brittle and rupturable by sound or pliant and porous, as well as a sound generator (296) directing sound against the microcapsules.

21. Appliance according to claim 20, characterised in that the microcapsules (298 to 302) are a mixture of different capsules which respond at different frequencies and/or different sound intensities, e.g. capsules with different wall materials, capsules with different diameters, capsules with different wall thicknesses.

22. Appliance according to claim 20 or 21, characterised in that the sound generator is a loudspeaker of a sound reproduction system.

23. Appliance according to claim 20 or 21, characterised in that the sound generator is an ultrasonic generator (296) which is disposed in the immediate vicinity of the substrate (274) bearing the microcapsules (298 to 302).

24. Appliance according to claim 22 or 23, characterised by a hard wall (278) disposed behind the substrate (274).

25. Appliance according to one of claims 20 to 24, characterised in that the substrate (274) is band-shaped and is moved past the sound generator (296) by a drive (284) according to the fragrance control signal.

26. Appliance according to one of claims 1 to 25, characterised in that there is controlled by the stimulus generator control signal at the same time a sound generator (34) whose output signal is mixed with the useful sound signal.

27. Appliance according to one of claims 1 to 26, characterised in that at least one (32) of the controllable stimulus generators cooperates with a coupling microphone (74) or a light detector, which responds to signals emitted simultaneously by a loudspeaker (18) or by an image generator and assigned to the control signals.

28. Appliance according to claim 27, characterised in that an impulse generator operating unit, the coupling microphone (74) and preferably also the stimulus generator (32) itself are accommodated in a common casing (72).

29. Appliance according to one of claims 1 to 28, characterised in that one (32) of the stimulus generators is a colour television set (90) which is impressed by means of a signal converter (92) with the control signals, which converts the control signals into video signals.

## Revendications

1. Appareil pour produire des sons et/ou des images, comprenant une source de signaux utiles (210, 224 ; 312, 314) destinée à des signaux de son et/ou d'image et pourvue d'une unité de sortie (226, 228 ; 318 à 322) des sons et/ou des images reliée à cette source, comprenant une source de signaux de commande (210, 224, 236 à 240 ; 306, 308, 236 à 240 ; 316, 324, 326, 236 à 240), et comprenant au moins un générateur de stimulations (244) commandé par cette source, les signaux de commande du générateur de stimulations présentant une faible vitesse de modification en comparaison des signaux utiles, caractérisé par le fait que la source de signaux de commande comprend : un générateur de signaux de commande (214, 224 ; 306, 308 ; 312 à 316) et une mémoire (236) destinée aux signaux de commande et raccordée à lui, ainsi qu'une unité de commande (220 ; 310 ; 326) au moyen de laquelle la mémoire (236) destinée aux signaux de commande peut être activée en vue de la mise en mémoire et de la lecture de signaux de commande ; et par le fait que la mémoire (236) destinée aux signaux de commande est une mémoire numérique, et que ses emplacements de mémoire sont adressés lors de la lecture par un compteur d'adressage (238) dont la borne de comptage (C) est reliée à la sortie d'une horloge lente spontanée (240), tandis que l'adressage de la mémoire (236) destinée aux signaux de commande a lieu, lors de la mise en mémoire des signaux de commande, au moyen de l'unité de commande (220 ; 326) et en fonction des signaux de commande à l'entrée.

2. Appareil selon la revendication 1, caractérisé par le fait que la source de signaux de commande (210, 224 ; 236 à 240 ; 306, 308, 236 à 240 ; 312 à 316, 236 à 240) excite une pluralité de générateurs de stimulations (244-i) par l'intermédiaire d'un décodeur de signaux de commande (242).

3. Appareil selon la revendication 1 ou 2, dans lequel la source de signaux utiles comprend une partie réceptrice d'un appareil de radio et/ou de télévision (312, 314, 318), caractérisé par le fait que la source de signaux de commande comprend un filtre de signaux de commande (316) qui filtre des signaux initiés en fonction de la fréquence ou du temps ou par des signaux de séparation, à partir du courant des signaux fournis par un circuit d'entrée (314) de la partie réceptrice de l'appareil de radio et/ou de télévision (314, 318).

4. Appareil selon la revendication 1 ou 2, dans lequel la source de signaux utiles comprend un support d'enregistrement de sons et/ou d'images dans lequel des pistes (216 ; 248) contenant des signaux de son et/ou d'image sont ménagées sur un matériau formant support (210 ; 246), caractérisé par le fait que des signaux de commande (214 ; 250) qui sont destinés à exciter le générateur de stimulations sont prévus en supplément sur le matériau formant support (210 ; 246).

5. Appareil selon l'une des revendications 1 à 4, destiné à un support d'enregistrement dans lequel les groupes de pistes de signaux de commande qui sont à chaque fois associés à l'un des groupes de commande (216-i) des signaux de son et/ou d'image sont enregistrés d'une manière contiguë dans une zone de pistes de signaux de commande (214), cependant que les groupes de pistes de signaux de commande destinés à des morceaux différents sont séparés par des signaux de séparation et sont aussi identifiés de préférence, caractérisé par le fait qu'un adressage partiel de la mémoire (236) destinée aux signaux de commande a lieu lors de la lecture par l'unité de commande (230 310) selon le numéro du morceau qu'il faut jouer à chaque fois.

6. Appareil selon l'une des revendications 1 à 5, caractérisé par le fait que le générateur de signaux de commande comporte un support de signaux de commande (308) et un appareil de lecture associé (306) ou une unité d'entrée de signaux de commande.

7. Appareil selon l'une des revendications 1 à 6, caractérisé par le fait que la source de signaux de commande comporte des moyens de couplage des phases (10, 22, 24 ; 42, 48 ; 52, 48) qui sont alimentés par le signal utile de son et/ou d'image et qui fournissent sur leur sortie le signal de commande du générateur de stimulations présentant la relation prédéterminée entre les phases par rapport au signal utile de son et/ou d'image.

8. Appareil selon la revendication 7, caractérisé par le fait que les moyens de couplage des phases comportent un circuit de filtrage (44) qui est alimenté par le signal de son, qui laisse passer par exemple les basses fréquences, dont l'entrée est alimentée par le signal utile de son et/ou d'image et dont la sortie fournit le signal de commande du générateur de stimulations.

9. Appareil selon la revendication 7, caractérisé par le fait que la source de signaux utiles est un instrument de musique comportant un diviseur de fréquence (22, 24) qui prédétermine le rythme d'une musique d'accompagnement, dont l'entrée est reliée à une horloge spontanée (10) et dont la sortie est reliée à un générateur de tonalité (34), et par le fait que les moyens de couplage de phase sont constitués par le diviseur de fréquence (22, 24) qui fournit le signal de commande du générateur de stimulations sur une sortie intermédiaire.

10. Appareil selon l'une des revendications 1 à 9, caractérisé par le fait que la source de signaux de commande comporte un diviseur de fréquence (22, 24) ou un multiplicateur de fréquence (48) qui est réglable, dont l'entrée est alimentée par le signal utile de son et/ou d'image et dont la sortie fournit le signal de commande du générateur de stimulations.

11. Appareil selon l'une des revendications 1 à 10, caractérisé par le fait que la source de signaux de commande comporte un générateur de courbes-enveloppes (30) dont le signal de sortie est amené à un circuit de commande (26) qui est alimenté sur une autre entrée par le signal de commande du générateur de stimulations, ce signal étant modulé en amplitude en fonction du signal de sortie engendré par le générateur de courbes-enveloppes (30).

12. Appareil selon l'une des revendications 1 à 11, caractérisé par le fait que l'un au moins des générateurs de stimulations pouvant être commandés est un générateur de parfum pouvant être commandé (56 à 62 ; 84 ; 244-i).

13. Appareil selon la revendication 12, caractérisé par le fait que l'un au moins des générateurs de parfum pouvant être commandés comprend un générateur de gaz porteur pouvant être commandé (56 à 60), ainsi qu'un volume de parfum (62) à travers lequel le gaz porteur barbote.

14. Appareil selon la revendication 13, caractérisé par le fait que le gaz porteur est de l'oxygène ou de l'air enrichi en oxygène.

15. Appareil selon l'une des revendications 12 à 14, caractérisé par le fait que l'un au moins des générateurs de parfum pouvant être commandés comprend : un porte-parfum (96) constitué par un support (98) et au moins un parfum en microcapsules déposé sur celui-ci, ainsi qu'un dispositif (104 à 112) pour briser les capsules qui peut être commandé et qui coopère successivement avec des zones différentes du porte-parfum (96).

16. Appareil selon la revendication 15, caractérisé par le fait que le porte-parfum (96) est en forme de bande, et qu'il est retiré d'un rouleau d'alimentation (94) par le dispositif pour briser les capsules qui comporte des rouleaux d'écrasement (108, 110).

17. Appareil selon l'une des revendications 12 à 16, caractérisé par le fait que l'un au moins des générateurs de parfum (244-i) pouvant être commandés comprend : un réservoir (262-i) qui peut être mis sous pression (264) et qui est rempli d'un liquide contenant au moins un parfum, un tube de pulvérisation (266) qui plonge dans le liquide contenant les parfums et une soupape de sortie (268) qui peut être commandée et qui est montée dans le tube de pulvérisation (266).

18. Appareil selon la revendication 17, caractérisé par le fait que plusieurs réservoirs (262-i) destinés à des liquides contenant des parfums sont reliés entre eux pour former un bloc de réservoirs.

19. Appareil selon la revendication 17 ou 18, caractérisé par le fait qu'un ventilateur (254) est disposé au voisinage des tubes de pulvérisation (266).

20. Appareil selon l'une des revendications 12 à 19, caractérisé par le fait que l'un au moins des générateurs de parfum (244-i) comprend : un substrat (274) et des microcapsules (298 à 302) qui sont disposées sur celui-ci et qui contiennent un parfum, la matière de la paroi des microcapsules étant fragile et pouvant être brisée par un son, ou étant élastique et poreuse, ainsi qu'un générateur de sons (296) qui dirige un son vers les microcapsules.

21. Appareil selon la revendication 20, caractérisé par le fait que les microcapsules (298 à 302) sont constituées par un mélange de capsules différentes qui répondent à une fréquence différente et/ou à une intensité différente du son, comme par exemple des capsules dont la matière de la paroi est différente, des capsules dont le diamètre est différent ou des capsules dont l'épaisseur de la paroi est différente.

22. Appareil selon la revendication 20 ou 21, caractérisé par le fait que le générateur de sons est un haut-parleur d'un dispositif de reproduction des sons.

23. Appareil selon la revendication 20 ou 21, caractérisé par le fait que le générateur de sons est un générateur d'ultrasons (296) disposé au voisinage immédiat du substrat (274) qui porte les microcapsules (298 à 302).

24. Appareil selon la revendication 22 ou 23, caractérisé par une paroi dure (278) qui est disposée derrière le substrat (274).

25. Appareil selon l'une des revendications 20 à 24, caractérisé par le fait que le substrat (274) est en forme de bande, et qu'il est déplacé devant le générateur de sons (296) par un entraînement (284) en fonction du signal de commande du parfum.

26. Appareil selon l'une des revendications 1 à 25, caractérisé par le fait que le signal de commande du générateur de stimulations commande en mène temps un générateur de tonalité (34) dont le signal de sortie est ajouté et mélangé au signal utile de son.

27. Appareil selon l'une des revendications 1 à 26, caractérisé par le fait que l'un au moins (32) des générateurs de stimulations pouvant être commandés coopère avec un microphone de couplage (74) ou avec un détecteur de lumière, respectivement, celui-ci répondant à des signaux qui sont émis par un haut-parleur (18) ou par un générateur d'images, respectivement, et qui sont associés aux signaux de commande.

28. Appareil selon la revendication 27, caractérisé par le fait qu'une unité de fonctionnement du générateur de stimulations, le microphone de couplage (74) et de préférence aussi le générateur de stimulations (32) lui-même sont logés dans un boîtier commun (72).

29. Appareil selon l'une des revendications 1 à 28, caractérisé par le fait que l'un (32) des générateurs de stimulations est un téléviseur couleur (90), celui-ci étant alimenté par les signaux de commande par l'intermédiaire d'un convertisseur de signaux (92) qui transforme les signaux de commande en signaux vidéo.
